(19) **Europäisches Patentamt European Patent Office Office européen des brevets**

(11) **EP 4 786 599 A1**

(12) **EUROPEAN PATENT APPLICATION**
published in accordance with Art. 153(4) EPC

(43) Date of publication:
05.08.2026 Bulletin 2026/32

(21) Application number: 24847258.1

(22) Date of filing: 26.09.2024

(51) International Patent Classification (IPC):
$C12N\ 15/62^{(2006.01)}$ $C12N\ 5/0783^{(2010.01)}$
$A61K\ 35/17^{(2025.01)}$ $A61P\ 35/00^{(2006.01)}$
$A61P\ 37/00^{(2006.01)}$

(52) Cooperative Patent Classification (CPC):
A61K 40/31; A61K 40/11; A61K 40/15;
A61K 40/4232; A61K 40/4234; A61K 40/4255;
C07K 16/2803; C07K 16/2878; C07K 16/30;
C07K 2317/622; C07K 2319/03

(86) International application number:
PCT/CN2024/121364

(87) International publication number:
WO 2025/067318 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.09.2023 US 202363540954 P

(71) Applicant: Pell Bio-Med Technology Co., Ltd.
Taipei City (TW)

(72) Inventors:
• HSU, Felix
Taipei City, Taiwan (TW)

• LIN, Wei-Chi
Taipei City, Taiwan (TW)
• WU, Wen-Ting
Taipei City, Taiwan (TW)
• LIN, Chen-Lung
Taipei City, Taiwan (TW)

(74) Representative: Cabinet Chaillot
16/20, avenue de l'Agent Sarre
B.P. 74
92703 Colombes Cedex (FR)

Remarks:
The complete document including Reference Table(s) and the Sequence Listing(s) can be downloaded from the EPO website

(54) **NUCLEIC ACID MOLECULE COMPRISING INTRACELLULAR SIGNALING DOMAIN AND NATURAL KILLER PROTEIN 30 TRANSMEMBRANE DOMAIN, AND CHIMERIC ANTIGEN RECEPTOR COMPRISING NUCLEIC ACID MOLECULE**

(57) Provided is an isolated nucleic acid molecule comprising an intracellular signaling domain and a NKp30 transmembrane domain, and a chimeric antigen receptor comprising the same. The isolated nucleic acid molecule comprising a nucleic acid sequences of (a) an extracellular antigen binding domain; (b) a hinge domain; (c) a NKp30 transmembrane domain; (d) a NKp30 cytoplasmic domain; and (e) an intracellular signaling domain. By introducing nucleic acid sequences of the intracellular signaling domain, the NKp30 transmembrane domain and the NKp30 cytoplasmic domain into a T cell, the resulting CAR-T cell is a multi-chain CAR-T cell with a NKp30 receptor complex, which forms stable immune synapses with cancer cells and exhibits excellent cytotoxicity against cancer cells.

**Description**

The present invention relates to a nucleic acid molecule for preparing a chimeric antigen receptor (CAR), particularly, a nucleic acid molecule for preparing CAR to exhibit excellent cytotoxicity against cancer cells. The present invention also relates to a CAR comprising the nucleic acid molecule and applications of the CAR comprising the nucleic acid molecule.

Cancer is caused by multi-stage transformation from normal cells to tumor cells and usually starts from a milder pre-cancerous lesion which gradually progresses to a malignant tumor, thereby leading to metastasis. The foresaid transformation is not caused by a single factor, but a result of interactions between individual genetic factors and external factors.

Cancer is the second leading cause of death in the world. According to the statistics from the World Health Organization in 2020, nearly ten million people die of cancer worldwide, accounting for one-sixth of the global deaths. Among all, the most common cancers in men are lung cancer, prostate cancer, colon cancer, gastric cancer and liver cancer; and the most common cancers in women are breast cancer, colorectal cancer, lung cancer, cervical cancer and thyroid cancer.

Cancer not only causes physical and mental pain to the patient, but also brings severe emotional and financial stress to the patient's family. However, in the high-stressed environment of the modern society, it is difficult to completely eliminate the adverse external factors, and the number of cancer patients still increases year by year.

Accordingly, there remains an urgent need for an effective treatment for cancer.

For overcoming the problems in the prior art, one objective or advantage of the present invention is to provide a novel chimeric antigen receptor (CAR) design for CAR-T cell, and when modified T cells are introduced into the human body, the modified T cells are able to identify tumor cells expressing specific antigen, followed by killing the tumor cells through cytotoxicity.

To achieve the foresaid objective, the present invention provides an isolated nucleic acid molecule comprising nucleic acid sequences encoding the following parts: (a) an extracellular antigen binding domain; (b) a hinge domain; (c) a Natural Killer Protein 30 (NKp30) transmembrane domain; (d) a NKp30 cytoplasmic domain; and (e) an intracellular signaling domain.

In the present invention, by introducing genes of the NKp30 transmembrane domain, the NKp30 cytoplasmic domain and the intracellular signaling domain into a T cell to make the T cell express a complex of NKp30 receptor and become a multi-chain CAR-T cell, the multi-chain CAR-T cell can form stable immune synapses with cancer cells, thereby exerting excellent cytotoxicity to the cancer cells.

Preferably, the foresaid extracellular antigen binding domain is a NKp30 extracellular domain.

Preferably, the foresaid extracellular antigen binding domain comprises a heavy chain variable region.

Preferably, the foresaid extracellular antigen binding domain comprises a heavy chain variable region of anti-mesothelin (MSLN) antibody, a heavy chain variable region of anti-CD22 antibody, a heavy chain variable region of anti-CD19 antibody, a heavy chain variable region of anti-BCMA (B-cell maturation antigen) antibody, a heavy chain variable region of anti-CD123 antibody, a heavy chain variable region of anti-GPRC5D (G protein-coupled receptor, class C, group 5, member D) antibody or a heavy chain variable region of anti-TSHR (thyroid-stimulating hormone receptor) antibody.

In one embodiment, the foresaid extracellular antigen binding domain may be selected from heavy chain variable regions derived from heavy chain antibodies (VHH) or a single-chain variable fragment (scFv); wherein, the said heavy chain variable regions of heavy chain antibodies may be derived from a single-chain binding region of heavy chain antibodies from Camelidae.

In one embodiment, the foresaid extracellular antigen binding domain may be a heavy chain variable region of anti-BCMA heavy chain antibody.

In one embodiment, the foresaid extracellular antigen binding domain may comprise a first heavy chain variable region of anti-BCMA heavy chain antibody and a second heavy chain variable region of anti-BCMA heavy chain antibody linked by $G_4S$, which is shown as the amino acid sequence of SEQ ID NO: 1; wherein, the first heavy chain variable region of anti-BCMA heavy chain antibody is shown as the amino acid sequence of SEQ ID NO: 2 and the second heavy chain variable region of anti-BCMA heavy chain antibody is shown as the amino acid sequence of SEQ ID NO: 3; wherein, the first heavy chain variable region of anti-BCMA heavy chain antibody comprises complementarity-determining regions (CDRs): CDRH1 is shown as the amino acid sequence of SEQ ID NO: 4; CDRH2 is shown as the amino acid sequence of SEQ ID NO: 5; and CDRH3 is shown as the amino acid sequence of SEQ ID NO: 6; and the second heavy chain variable region of anti-BCMA heavy chain antibody comprises the following CDRs: CDRH1 is shown as the amino acid sequence of SEQ ID NO: 7; CDRH2 is shown as the amino acid sequence of SEQ ID NO: 8; and CDRH3 is shown as the amino acid sequence of SEQ ID NO: 9.

In one embodiment, the foresaid extracellular antigen binding domain may comprise the heavy chain variable region of anti-CD19 antibody. The heavy chain variable region of anti-CD19 antibody comprises CDRs: CDRH1 is shown as the amino acid sequence of SEQ ID NO: 10; CDRH2 is shown as the amino acid sequence of SEQ ID NO: 11; and CDRH3 is shown as the amino acid sequence of SEQ ID NO: 12. In one embodiment, the foresaid extracellular antigen binding domain further comprises a light chain variable region of anti-CD19 antibody, and the light chain variable region of anti-CD19 antibody comprises the following CDRs: CDRL1 is shown as the amino acid sequence of SEQ ID NO: 13; CDRL2 is shown as the amino acid sequence of SEQ ID NO: 14; and CDRL3 is shown as the amino acid sequence of SEQ ID NO: 15. In one embodiment, the foresaid extracellular antigen binding domain is FMC63 scFv and is shown as the amino acid sequence of SEQ ID NO: 16.

In one embodiment, the foresaid extracellular antigen binding domain comprises the heavy chain variable region of anti-mesothelin antibody, and the heavy chain variable region of anti-mesothelin antibody comprises the following CDRs: CDRH1 is shown as the amino acid sequence of SEQ ID NO: 17; CDRH2 is shown as the amino acid sequence of SEQ ID NO: 18; and CDRH3 is shown as the amino acid sequence of SEQ ID NO: 19. In one embodiment, the foresaid extracellular antigen binding domain further comprises a light chain variable region of anti-mesothelin antibody, and the light chain variable region of anti-mesothelin antibody comprises the following CDRs: CDRL1 is shown as the amino acid sequence of SEQ ID NO: 20; CDRL2 is shown as the amino acid sequence of SEQ ID NO: 21; and CDRL3 is shown as the amino acid sequence of SEQ ID NO: 22. In one embodiment, the foresaid extracellular antigen binding domain is SS1 scFv and is shown as the amino acid sequence of SEQ ID NO: 23.

In one embodiment, the foresaid extracellular antigen binding domain comprises the heavy chain variable region of anti-BCMA antibody, and the heavy chain variable region of anti-BCMA antibody comprises the following CDRs: CDRH1 is shown as the amino acid sequence of SEQ ID NO: 24; CDRH2 is shown as the amino acid sequence of SEQ ID NO: 25; and CDRH3 is shown as the amino acid sequence of SEQ ID NO: 26. In one embodiment, the foresaid extracellular antigen binding domain further comprises a light chain variable region of anti-BCMA antibody, and the light chain variable region of anti-BCMA antibody comprises the following CDRs: CDRL1 is shown as the amino acid sequence of SEQ ID NO: 27; CDRL2 is shown as the amino acid sequence of SEQ ID NO: 28; and CDRL3 is shown as the amino acid sequence of SEQ ID NO: 29. In one embodiment, the foresaid extracellular antigen binding domain is J6M0 scFv and is shown as the amino acid sequence of SEQ ID NO: 30. In another embodiment, the heavy chain variable region of anti-BCMA antibody comprises the following CDRs: CDRH1 is shown as the amino acid sequence of SEQ ID NO: 31; CDRH2 is shown as the amino acid sequence of SEQ ID NO: 32; and CDRH3 is shown as the amino acid sequence of SEQ ID NO: 33. In one embodiment, the foresaid extracellular antigen binding domain further comprises a light chain variable region of anti-BCMA antibody, and the light chain variable region of anti-BCMA antibody comprises the following CDRs: CDRL1 is shown as the amino acid sequence of SEQ ID NO: 34; CDRL2 is shown as the amino acid sequence of SEQ ID NO: 35; and CDRL3 is shown as the amino acid sequence of SEQ ID NO: 36. In one embodiment, the foresaid extracellular antigen binding domain is BB2121 scFv and is shown as the amino acid sequence of SEQ ID NO: 37.

In one embodiment, the foresaid extracellular antigen binding domain comprises the heavy chain variable region of anti-CD22 antibody, and the heavy chain variable region of anti-CD22 antibody comprises the following CDRs: CDRH1 is shown as the amino acid sequence of SEQ ID NO: 38; CDRH2 is shown as the amino acid sequence of SEQ ID NO: 39; and CDRH3 is shown as the amino acid sequence of SEQ ID NO: 40. In one embodiment, the foresaid extracellular antigen binding domain further comprises a light chain variable region of anti-CD22 antibody, and the light chain variable region of anti-CD22 antibody comprises the following CDRs: CDRL1 is shown as the amino acid sequence of SEQ ID NO: 41; CDRL2 is shown as the amino acid sequence of SEQ ID NO: 42; and CDRL3 is shown as the amino acid sequence of SEQ ID NO: 43. In one embodiment, the foresaid extracellular antigen binding domain is m971 scFv and is shown as the amino acid sequence of SEQ ID NO: 44. In another embodiment, the foresaid extracellular antigen binding domain may comprise the heavy chain variable region of anti-CD22 antibody, and the heavy chain variable region of anti-CD22 antibody comprises the following CDRs: CDRH1 is shown as the amino acid sequence of SEQ ID NO: 45; CDRH2 is shown as the amino acid sequence of SEQ ID NO: 46; and CDRH3 is shown as the amino acid sequence of SEQ ID NO: 47. In one embodiment, the foresaid extracellular antigen binding domain further comprises a light chain variable region of anti-CD22 antibody, and the light chain variable region of anti-CD22 antibody comprises the following CDRs: CDRL1 is shown as the amino acid sequence of SEQ ID NO: 48; CDRL2 is shown as the amino acid sequence of SEQ ID NO: 49; and CDRL3 is shown as the amino acid sequence of SEQ ID NO: 50. In one embodiment, the foresaid extracellular antigen binding domain is LL2 scFv and is shown as the amino acid sequence of SEQ ID NO: 51. In another embodiment, the foresaid extracellular antigen binding domain comprises the heavy chain variable region of anti-CD22 antibody, and the heavy chain variable region of anti-CD22 antibody comprises the following CDRs: CDRH1 is shown as the amino acid sequence of SEQ ID NO: 52; CDRH2 is shown as the amino acid sequence of SEQ ID NO: 53; and CDRH3 is shown as the amino acid sequence of SEQ ID NO: 54. In one embodiment, the foresaid extracellular antigen binding domain further comprises a light chain variable region of anti-CD22 antibody, and the light chain variable region of anti-CD22 antibody comprises the following CDRs: CDRL1 is shown as the amino acid sequence of SEQ ID NO: 55; CDRL2 is shown as the amino acid sequence of SEQ ID NO: 56; and CDRL3 is shown as the amino acid sequence of SEQ ID NO: 57. In one embodiment, the foresaid extracellular antigen binding domain is G5/44 scFv and is shown as the amino acid sequence of SEQ ID NO: 58. In another embodiment, the foresaid extracellular antigen binding domain comprises the heavy chain variable region of anti-CD22 antibody, and the heavy chain variable region of anti-CD22 antibody comprises the following CDRs: CDRH1 is shown as the amino acid sequence of SEQ ID NO: 59; CDRH2 is shown as the amino acid sequence of SEQ ID NO: 60; and CDRH3 is shown as the amino acid sequence of SEQ ID NO: 61. In one embodiment, the foresaid extracellular antigen binding domain further comprises a light chain variable region of anti-CD22 antibody, and the light chain variable region of anti-CD22 antibody comprises the following CDRs: CDRL1 is shown as the amino acid sequence of SEQ ID NO: 62; CDRL2 is shown as the amino acid sequence of SEQ ID NO: 63; and CDRL3 is shown as the amino acid sequence of SEQ ID NO: 64. In one embodiment, the foresaid extracellular antigen binding domain is RFB4 scFv and is shown as the amino acid sequence of SEQ ID NO: 65.

Preferably, the foresaid extracellular antigen binding domain comprises the heavy chain variable region of anti-CD19 antibody, and may be FMC63 scFv.

Preferably, the foresaid extracellular antigen binding domain comprises the heavy chain variable region of anti-mesothelin antibody, and may be SS1 scFv.

Preferably, the foresaid extracellular antigen binding domain comprises the heavy chain variable region of anti-BCMA antibody, and may be J6M0 scFv or BB2121 scFv.

Preferably, the foresaid extracellular antigen binding domain comprises the heavy chain variable region of anti-CD22 antibody, and may be m971 scFv, LL2 scFv, G5/44 scFv or RFB4 scFv.

Preferably, the foresaid hinge domain is selected from the group consisting of: $(G_4S)_3$ (shown as SEQ ID NO: 66), a hinge domain of CD8 (shown as SEQ ID NO: 67), a hinge domain of CD28 (shown as SEQ ID NO: 68), a hinge domain of IgG4 (shown as SEQ ID NO: 69), EAAAKGGGGS (shown as SEQ ID NO: 70), $(EAAAK)_3$ (shown as SEQ ID NO: 71), a hinge domain of GST (shown as SEQ ID NO: 72), a hinge domain of EAAAK-GS (shown as SEQ ID NO: 73), a hinge domain of IgD (shown as SEQ ID NO: 74), an IgG4-CH3 domain (shown as SEQ ID NO: 75) and $(AP)_6$ (shown as SEQ ID NO: 76).

Preferably, the foresaid isolated nucleic acid molecule may further comprise a nucleic acid sequence of an adapter gene. More preferably, the said adapter may be CD3ζ, DAP10, CD79, FcεR1$\gamma$, FcR$\gamma$ or Fcβ/$\gamma$. The present invention adopts the full-length protein of CD3ζ, DAP10, CD79, FcεR1$\gamma$, FcR$\gamma$ or Fcβ/$\gamma$ as the adapter.

Preferably, the foresaid intracellular signaling domain is intracellular signaling domains of DAP12, 41BB or CD28. More preferably, the foresaid intracellular signaling domain is an intracellular signaling domain of DAP12.

Preferably, the foresaid hinge domain and the NKp30 transmembrane domain are linked by a NKp30 extracellular stalk domain.

Preferably, the foresaid isolated nucleic acid molecule may further comprise a nucleic acid sequence of signal transduction domain of CD3ζ, a nucleic acid sequence of FcεR1$\gamma$ adapter or a nucleic acid sequence encoding T2A self-cleaving peptide. For example, the nucleic acid sequence of FcεR1$\gamma$ adapter may be located at 5' end of the foresaid isolated nucleic acid molecule; and the nucleic acid sequence of T2A self-cleaving peptide may be located between the nucleic acid sequence of intracellular signaling domain and the nucleic acid sequence of the $\gamma$ NKp30 transmembrane domain, or located between the nucleic acid sequence of FcεR1 adapter and the nucleic acid sequence of the NKp30 transmembrane domain.

The hinge domain and the NKp30 transmembrane domain are linked by a NKp30 extracellular stalk domain.

Preferably, in the foresaid isolated nucleic acid molecule, when the extracellular antigen binding domain comprises a heavy chain variable region of anti-CD19 antibody, the hinge domain is the hinge domain of IgG4. When the extracellular antigen binding domain is combined with the hinge domain, it can make the produced lentivirus have high transduction efficiency, and the produced CAR-T cells have better expansion rates, higher proportions of stem cell-like memory T-cells (Tscm) and exhibit stronger cytotoxicity. More preferably, the foresaid heavy chain variable region of anti-CD19 antibody is a fragment of FMC63 scFv.

Preferably, in the foresaid isolated nucleic acid molecule, when the extracellular antigen binding domain comprises a heavy chain variable region of anti-mesothelin antibody, the hinge domain is the hinge domain of $(G_4S)_3$. When the extracellular antigen binding domain is combined with the hinge domain, it can make the produced lentivirus have high transduction efficiency, and the produced CAR-T cells have better expansion rates, higher proportions of Tscm and exhibit stronger cytotoxicity. More preferably, the foresaid heavy chain variable region of anti-mesothelin antibody is a fragment of SS1 scFv.

Preferably, in the foresaid isolated nucleic acid molecule, when the extracellular antigen binding domain comprises a heavy chain variable region of anti-BCMA antibody, the hinge domain is the hinge domain of CD28. When the extracellular antigen binding domain is combined with the hinge domain, it can make the produced lentivirus have high transduction efficiency, and the produced CAR-T cells have better expansion rates, higher proportions of Tscm and exhibit stronger cytotoxicity. More preferably, the foresaid heavy chain variable region of anti-BCMA antibody is a fragment of J6M0 scFv.

Preferably, in the foresaid isolated nucleic acid molecule, when the extracellular antigen binding domain comprises a heavy chain variable region of anti-CD22 antibody, the hinge domain is the hinge domain of CD28. When the extracellular antigen binding domain is combined with the hinge domain, it can make the produced lentivirus have high transduction efficiency, and the produced CAR-T cells have better expansion rates, higher proportions of Tscm and exhibit stronger cytotoxicity. More preferably, the foresaid heavy chain variable region of anti-CD22 antibody is a fragment of m971 scFv.

To achieve the foresaid objective, the present invention further provides a plasmid, which comprises the foresaid isolated nucleic acid molecule, and the plasmid may be a DNA plasmid or an RNA plasmid. In accordance with the present invention, the sequential order of $\gamma$ nucleic acid sequences from 5' end to 3' end of the plasmid is FcεR1 adapter, the intracellular signaling domain, T2A self-cleaving peptide, the extracellular antigen binding domain, the hinge domain, the NKp30 transmembrane domain and the NKp30 cytoplasmic domain. In one embodiment, the sequential order of nucleic acid sequences from 5' end to 3' end of the plasmid is 5' LTR, promoter, FcεR1 $\gamma$ adapter, the intracellular signaling domain, T2A self-cleaving peptide, the extracellular antigen binding domain, the hinge domain, the NKp30 transmembrane domain, the NKp30 cytoplasmic domain and 3' LTR.

To achieve the foresaid objective, the present invention further provides a method for preparing a chimeric antigen receptor cell, which comprises introducing the foresaid isolated nucleic acid molecule into a nucleated cell.

Preferably, in the foresaid method for preparing a chimeric antigen receptor cell, the said "introducing" is achieved by lentivirus transduction.

Preferably, the foresaid nucleated cell is leukocytes.

Preferably, the foresaid nucleated cell is T cells, nature killer cells, nature killer T cells or adipose-derived stem cells.

To achieve the foresaid objective, the present invention further provides an isolated cell, which comprises the foresaid isolated nucleic acid molecule.

Preferably, the foresaid isolated cell is a T cell, a natural killer cell, a natural killer T cell or an adipose-derived stem cell (ADSC).

To achieve the foresaid objective, the present invention further provides a use of the foresaid isolated cell to prepare a medication for treating or alleviating cancer, for example, treating B-cell acute lymphoblastic leukemia, B-cell lymphoma, Leydig cell tumor, ovarian cancer, acute myeloid leukemia, mesothelioma, colon cancer, lung cancer, pancreatic cancer, thyroid cancer and/or multiple myeloma.

To achieve the foresaid objective, the present invention further provides a use of the foresaid isolated cell to prepare a medication for treating or alleviating an autoimmune disease; wherein, the said autoimmune disease may be a disease related to autoimmune reaction that can be treated by CD19 CAR-T cell. In one embodiment, the foresaid autoimmune disease is systemic lupus erythematosus (SLE), idiopathic inflammatory myositis, systemic sclerosis or multiple sclerosis.

The advantages of the present invention are to introduce nucleic acid sequences of the NKp30 transmembrane domain, the NKp30 cytoplasmic domain and the intracellular signaling domain into a T cell to make the T cell express a complex of NKp30 receptor and become a multi-chain CAR-T cell, which can form stable immune synapses with cancer cells, thereby having excellent cytotoxicity to cancer cells. And the produced CAR-T cells have excellent cytotoxicity both *in vivo* and *in vitro*, do not over-activate T cells when significantly killing cancer cells through cytotoxicity, show persistency in re-challenge assay, and possess high proportions of Tscm, and it is expected to display reduced cytokine release syndrome and neurotoxicity caused by CAR-T cell therapy.

In the drawings:

FIG.1 is a schematic illustration of the pLAS5w vector.
FIG.2 is a schematic illustration of plasmids for Examples 1-1A to 1-2A and Comparative Example 1A.
FIG.3 is a schematic illustration of cell membrane protein structures for CAR-T cells of Examples 1-1C, 1-2C and Comparative Example 1C.
FIGs.4A to 4D are the results of the distribution of T cell subpopulations from flow cytometry of CAR-T cells of Examples 1-1C, 1-2C and Comparative Example 1C, and the un-transduced group.
FIGs.5A, 5B and 5C respectively are the comparison results of real-time cytotoxicity of CAR-T cells of Examples 1-1C and 1-2C, CAR-T cells of Comparative Example 1C, T cells of the un-transduced group, and the target cells at E:T ratios of 10:1, 3:1 and 1:1.
FIG.6 is a schematic illustration of plasmids for Example 2A and Comparative Example 2A.
FIG.7 is schematic illustrations of cell membrane protein structures for CAR-T cells of Example 2C and Comparative Example 2C.

FIGs.8A and 8B respectively are the results of anti-mesothelin antibody and NKp30 expressions of CAR-T cells of Example 2C from flow cytometry in Test Example 8.

FIGs.8C and 8D respectively are the results of anti-mesothelin antibody and NKp30 expressions of CAR-T cells of Comparative Example 2C from flow cytometry in Test Example 8.

FIGs.9A, 9B and 9C respectively are the results of the distribution of T cell subpopulations on the 7th day from flow cytometry of Example 2C, Comparative Example 2C and the un-transduced group in Test Example 9.

FIGs.10A, 10B, 10C and 10D respectively are the comparison results of real-time cytotoxicity of CAR-T cells of Example 2C, CAR-T cells of Comparative Example 2C, T cells of the un-transduced group, and the target cells at E:T ratios of 3:1, 1:1, 0.3:1 and 0.1:1 in Test Example 10.

FIG.11A is the *in vivo* anti-tumor effects of CAR-T cells of Example 2C, CAR-T cells of Comparative Example 2C, and T cells of the un-transduced group at E:T ratios of 3:1 and 1:1 in Test Example 12.

FIG.11B is the logarithmic signals of bioluminescence imaging of CAR-T cells of Example 2C, CAR-T cells of Comparative Example 2C, T cells of the un-transduced group, and the saline group at E:T ratios of 3:1 and 1:1 in Test Example 12.

FIG.12A is the *in vivo* anti-tumor re-challenge assay results of CAR-T cells of Example 2C, CAR-T cells of Comparative Example 2C, and the saline group at E:T ratios of 3:1 and 1:1 in Test Example 12.

FIG.12B is the logarithmic signals of bioluminescence imaging for the *in vivo* anti-tumor re-challenge assay of CAR-T cells of Example 2C, CAR-T cells of Comparative Example 2C, and the target cells at E:T ratios of 3:1 and 1:1 in Test Example 12; wherein * represents p-value <0.05, and ** represents p-value <0.01.

FIG.13A is ratios of T cells in peripheral blood of mice in each group in Test Example 12.

FIG.13B is ratios of CAR-T cells in T cells of mice in each group in Test Example 12.

FIG.14 is a schematic illustration of plasmids for Example 3A, Comparative Example 3-1A and Comparative Example 3-2A.

FIG.15 is a schematic illustration of cell membrane protein structures for CAR-T cells of Example 3C, Comparative Example 3-1C and Comparative Example 3-2C.

FIGs.16A and 16B respectively are the results of anti-CD22 receptor and NKp30 expressions of CAR-T cells of Example 3C from flow cytometry in Test Example 15.

FIGs.16C and 16D respectively are the results of anti-CD22 receptor and NKp30 expressions of CAR-T cells of Comparative Example 3-1C from flow cytometry in Test Example 15.

FIGs.17A, 17B, 17C and 17D respectively are the results of the distribution of T cell subpopulations on the 7th day from flow cytometry of Example 3C, Comparative Example 3-1C, Comparative Example 3-2C and the un-transduced group in Test Example 16.

FIGs.18A and 18B are the comparison results of real-time cytotoxicity of CAR-T cells of Example 3C, CAR-T cells of Comparative Example 3-1C and Comparative Example 3-2C and T cells of the un-transduced group at E:T ratios of 3:1 and 1:1 in Test Example 17.

FIG.19 is the comparison results of cytotoxicity from flow cytometry of CAR-T cells of Example 3C, CAR-T cells of Comparative Example 3-1C and Comparative Example 3-2C, and T cells of the un-transduced group at E:T ratios of 3:1 and 1:1in Test Example 18.

FIG.20 is schematic illustration of plasmids for Example 4A and Comparative Example 4A.

FIG.21 is schematic illustration of cell membrane protein structures for CAR-T cells of Example 4C and Comparative Example 4C.

FIGs.22A and 22B respectively are the results of anti-CD19 receptor and NKp30 expressions of CAR-T cells of Example 4C from flow cytometry in Test Example 21.

FIGs.22C and 22D respectively are the results of anti-CD19 receptor and NKp30 expressions of CAR-T cells of Comparative Example 4C from flow cytometry in Test Example 21.

FIGs.23A, 23B and 23C respectively are the results of the distribution of T cell subpopulations on the 7th day from flow cytometry of Example 4C, Comparative Example 4C, and the un-transduced group in Test Example 22.

FIG.24 is the comparison results of real-time cytotoxicity of CAR-T cells of Example 4C, CAR-T cells of Comparative Example 4C, T cells of the un-transduced group, the target cells and the whole lysis group at an E:T ratio of 1:1 in Test Example 23.

FIG.25 is the comparison results of luciferase-based cytotoxicity of CAR-T cells of Example 4C, CAR-T cells of Comparative Example 4C and T cells of the un-transduced group at E:T ratios of 3:1 and 1:1.

The following embodiments are used to further explain the present invention, and the embodiments do not limit the foresaid disclosures of the present invention. One person skilled in the art can make some modifications and variations without departing from the scope of the invention.

**I. Preparation and test of CAR-T cells expressing intracellular signaling domain and NKp30 fragment**

**Example 1-1: A nucleic acid molecule comprising the DAP12 intracellular signaling domain and the full-length NKp30**

First, the 3' end of a nucleic acid sequence of full length FcεR1ɣ (shown as SEQ ID NO: 77) was linked to the 5' end of a nucleic acid sequence of DAP12 intracellular signaling domain (shown as SEQ ID NO: 78), to obtain a linked nucleic acid molecule fragment 1. Then, the linked nucleic acid molecule fragment 1 was linked to a sequence of NKp30 complete fragment (comprising immunoglobulin-like domain) (shown as SEQ ID NO: 79) by a nucleic acid sequence of T2A self-cleaving peptide (shown as SEQ ID NO: 80), to obtain a nucleic acid molecule having DAP12 intracellular signaling domain and NKp30 complete fragment (shown as SEQ ID NO: 81) of Example 1-1. Wherein, the NKp30 complete fragment comprised an immunoglobulin-like domain, a stalk domain, a NKp30 transmembrane (Tm) domain and a NKp30 cytoplasmic domain. The nucleic acid molecule having DAP12 intracellular signaling domain and NKp30 complete fragment of Example 1-1 was, from the 5' end to the 3' end, the nucleic acid sequence of full length FcεR1

.1ɣ, the nucleic acid sequence of DAP12 intrace

ce of T2A self-cleaving peptide and NKp30 cor

acid molecule comprising the 41BB intrace

:p30

Example 1-2 was similar to the preparation metl

racellular signaling domain was 41BB intrace

82). The obtained nucleic acid molecule having

0 complete fragment of Example 1-2 was sho

, the nucleic acid sequence of DAP12 intracellular signaling domain, the nucleic acid sequence of T2A self-cleaving peptide and NKp30 complete fragment.

**Example 1-2: A nucleic acid molecule comprising the 41BB intracellular signaling domain and the full-length NKp30**

The preparation method of Example 1-2 was similar to the preparation method of Example 1-1, except that the used intracellular signaling domain was 41BB intracellular signaling domain (shown as SEQ ID NO: 82). The obtained nucleic acid molecule having 41BB intracellular signaling domain and NKp30 complete fragment of Example 1-2 was shown as SEQ ID NO: 83.

**Comparative Example 1: modified wild-type NKp30 nucleic acid molecule**

First, the 3' end of the nucleic acid sequence of full length FcεR1ɣ (shown as SEQ ID NO: 77) was linked to the 5' end of the foresaid NKp30 complete fragment (shown as SEQ ID NO: 79) by the nucleic acid sequence of T2A self-cleaving peptide (shown as SEQ ID NO: 80), to obtain the modified wild-type NKp30 nucleic acid molecule of Comparative Example 1 (shown as SEQ ID NO: 84). The modified wild-type NKp30 nucleic acid molecule of Comparative Example1 was, from the 5' end to the 3' end, the nucleic acid sequence of full length FcεR1ɣ, the nucleic acid sequence of T2A self-cleaving peptide and NKp30 complete fragment.

**Examples 1-1A, 1-2A and Comparative Example 1A: Construction of plasmids with the nucleic acid molecules of Examples 1-1, 1-2 or Comparative Example 1**

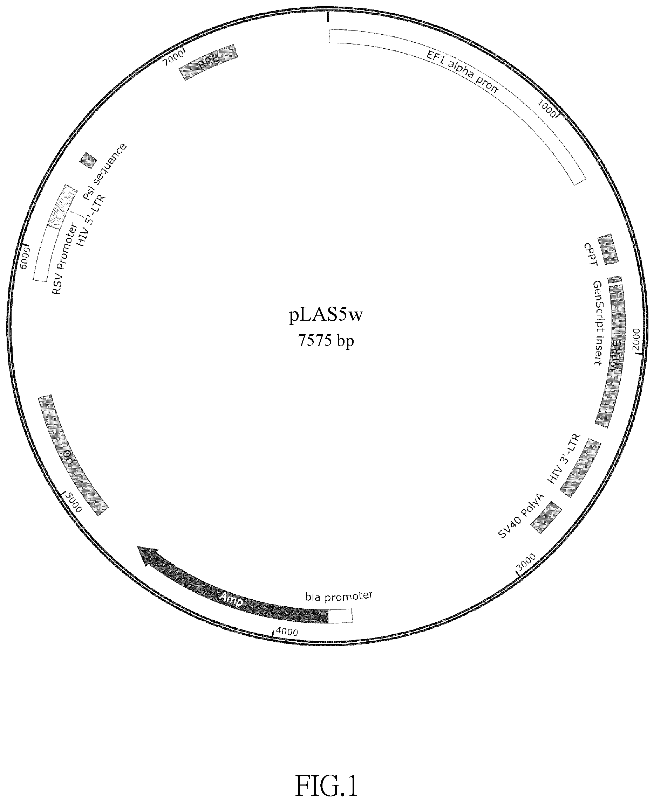

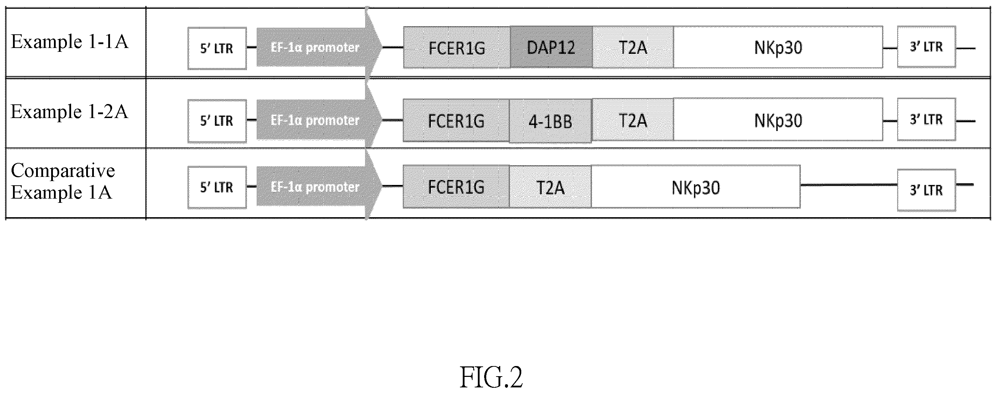

As shown in FIG.1, the pLAS5w.Ppuro vector having a nucleotide sequence of TAR chimeric 5' LTR and driven by RSV promoter was purchased from Academia Sinica RNAi Core facility, Product No.: C6-8-39, and after hPGK promoter and PAC gene thereof were deleted (conducted by GenScript), a pLAS5w vector was obtained. The nucleic acid molecules of Examples 1-1, 1-2 and Comparative Example 1 were ligated into the pLAS5w vector by using restriction enzymes of BstBI and EcoRI, and the plasmids of Examples 1-1A and 1-1B and a plasmid of Comparative Example 1A were obtained and are shown in FIG.2.

**Examples 1-1B and 1-2B and Comparative Example 1B: Preparation of lentiviruses with the nucleic acid molecules of Examples 1-1, 1-2 or Comparative Example 1**

Lentivirus particles of Examples 1-1B and 1-2B and Comparative Example 1B were produced by transfecting human embryonic kidney cells 293 (HEK 293 cells, ATCC, CRL-3216) with target genes using Polyjet transfection reagent (SignaGen Laboratories, SL100688). Specifically, 50 $\mu$g of one of the plasmids of Examples 1-1A and 1-2A or Comparative Example 1A comprising the target genes was adopted as the transfer plasmid; 45 $\mu$g of pCMV deltaR8.91 (purchased from Academia Sinica) was adopted as the packaging plasmid; and 5 $\mu$g of pMD.G (purchased from Academia Sinica) was adopted as the envelope plasmid. Then, the foresaid plasmids were incubated with 300 $\mu$L of Polyjet transfection reagent in 10 mL of serum-free DMEM for 15 minutes to obtain a Polyjet/DNA mixture. Then, the Polyjet/DNA mixture was added into 100 mL of DMEM with 10% FBS, and then added dropwise into a T875 flask that was previously seeded with $9\times10^7$ HEK293 cells. After culturing for 12 to 18 hours, the medium was replaced with 100 mL of Opti-MEM (Gibco, 31985070) containing 1 mM of sodium pyruvate (Sartorius). After transfecting for 16 hours, the medium was replaced with fresh Opti-MEM. After transfecting for 48 hours, the first supernatant containing lentivirus particles was collected, and the medium was then replenished with fresh Opti-MEM containing 1 mM of sodium pyruvate for 24 hours culture. The supernatant containing lentivirus particles was collected again, and the supernatant containing lentivirus particles was centrifuged at 1500 rpm, 4°C for 10 minutes to remove cell debris. Then, the supernatants were filtered by a filter of 0.45 $\mu$m pore size (Membrane-solutions). The two collected and filtered supernatants were combined and then concentrated by Lenti-X reagent (Takara Bio) at a ratio of 3:1, *i.e.*, the volume ratio of the filtered supernatant and Lenti-X reagent was 3:1. Specifically, the supernatant and Lenti-X concentration reagent were mixed by rotation upside down at 4°C overnight, and then centrifuged at 1500 rpm, 4°C for 45 minutes. The supernatant was removed, and the pellet was resuspended in 2 mL of X-VIVO™ 15 medium (Lonza) to obtain concentrated lentiviruses of Examples 1-1B and 1-2B and concentrated lentivirus of Comparative Example 1B, which were stored at -80°C for backup.

**Test Example 1: Titer determination of lentiviruses of Examples 1-1B and 1-2B and Comparative Example 1B**

Viral titers were determined by transducing $4\times10^4$ Jurkat cells (T cells of human acute leukemia, purchased from BCRC, Cat. No. 60424) using lentivirus samples that were 3-fold serial diluted, wherein, the lentivirus samples used in Test Example 1 were the concentrated lentiviruses of Examples 1-1B and 1-2B and the concentrated lentivirus of Comparative Example 1B. 50 $\mu$L of lentivirus sample was added into 100 $\mu$L of X-VIVO™ 15 medium, which was subjected to 3-fold serial dilution with the above-mentioned medium, and this dilution step was repeated until 6561-fold dilution, thereby obtaining serval groups of series-diluted lentivirus samples. Next, 50 $\mu$L of each series-diluted lentivirus sample was added into a 96-well round-bottom plate, and each well was $4\times10^4$ Jurkat cells/100 $\mu$L/well. After the lentivirus was added, the lentiviral transduction was performed for 72 hours. After excess lentivirus was washed out, anti-NKp30 antibody labeled with BV421 fluorescent dye (BioLegend, Cat. No. 325228) with a concentration of 1 $\mu$g/mL was adopted for detecting the expression of the NKp30. A flow cytometer (Sony, SA3800) was used for analyzing the expression of the target protein, thereby determining the transduction efficiency of lentivirus. Besides, un-transduced Jurkat cells were used as a negative control. The functional viral titer of lentivirus with the nucleic acid molecule of intracellular signaling domain and NKp30 complete fragment (comprising immunoglobulin-like domain) was calculated by the following equation, and the obtained functional viral titers were then used to determine the amount of lentivirus to be applied for CAR-T cell preparation.

Functional Titer of Lentivirus [transducing unit (TU) / mL] = (percentage of cells expressing NKp30) $\times$ $4\times10^4$ (cells) $\times$ 20 $\times$ dilution Fold.

The experimental results were shown in the following Table 1.

Table 1: the functional viral titers (transducing unit, TU / mL) of lentiviruses of Examples 1-1B and 1-2B and Comparative Example 1B

| Group | Example 1-1B | Example 1-2B | Comparative Example 1B |
|---|---|---|---|
| Functional viral titer (TU/mL) | $>1\times10^9$ | $>1\times10^9$ | $>1\times10^9$ |

When the functional viral titer was more than or equal to $1\times10^6$ TU/mL, T cells can be successfully transduced and become CAR-T cells with a lower amount of lentivirus, whereas, a lower volume of lentivirus used during transduction tended to correlate with better growth of T cells for CAR-T cells production. Therefore, according to the results in Table 1, Example 1-1B, Example 1-2B and Comparative Example 1B met the foresaid criteria, which further benefited preparation of CAR-T cells.

**Examples 1-1C and 1-2C and Comparative Example 1C: Preparing CAR-T cells with the nucleic acid molecules of Examples 1-1 and 1-2 or Comparative Example 1**

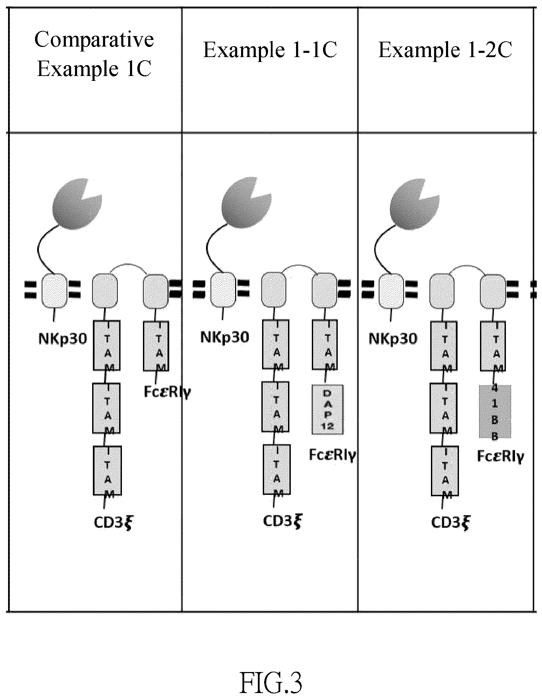

Fresh peripheral blood samples were collected from healthy volunteers under their consent. On day 0, human peripheral blood mononuclear cells (PBMCs) were isolated using SepMate centrifuge tubes (STEMCELL, Cat. No. 86450). In brief, the fresh blood samples were diluted with the same volume of PBS buffer containing 2% FBS to obtain diluted blood samples. Next, the diluted blood samples were added dropwise along tube wall of the SepMate centrifuge tube, during which the SepMate centrifuge tube remained in a vertical position, to mix the blood sample and Ficoll density-gradient centrifugation solution, followed by centrifugation at $1200 \times g$ at room temperature for 20 minutes. Next, the supernatant (rich in mononuclear cells) was decanted into a new tube and repeatedly washed by PBS buffer containing 2% FBS, followed by centrifugation at $300 \times g$ at room temperature for 8 minutes to collect the lower part of PBMC. Next, Dynabeads coated with anti-CD3/CD28 antibodies, *i.e.*, CTS (Cell Therapy Systems) Dynabeads CD3/CD28 (Gibco, Cat. No. 40203D); in combination with DynaMag-Spin (purchased from Thermo) were used to isolate T cells from the collected PBMCs. The proportion of CD3$^+$ T cells in the PBMC was then measured by anti-CD3 antibody (Biolegend, Cat.No.300441) detection and flow cytometric analysis, and the quantity of the CD3$^+$ T cells was calculated. With quantity three times than the CD3$^+$ T cells, Dynabeads coated with anti-CD3/CD28 antibodies were added into the PBMC, and then incubated at room temperature for 30 to 60 minutes to allow Dynabeads coated with anti-CD3/CD28 antibodies to bind to T cells. Next, DynaMag-Spin (Thermo) was used to select for CD3-positive T cells with the removal of B cells, NK cells and mononuclear cells in the PBMC, and then complexes of Dynabeads and T cells formed by antigen-antibody binding were thereby obtained. The complexes of Dynabeads and T cells with a density of $2 \times 10^5$ cells/mL were then dispersed into X-VIVO$^{TM}$ 15 medium (Lonza) containing 200 IU/mL of recombinant human IL-2 (R&D Systems), and then cultured at 37°C in 5% $CO_2$ for 24 hours to activate the T cells. Next, one of lentiviruses of Examples 1-1B and 1-2B or Comparative Example 1B were respectively added to the activated T cells, at a multiplicity of infection (MOI) of 5 and left for 24 hours for lentiviral transduction; thereby obtaining complexes of Dynabeads and CAR-T cells of Examples 1-1C and 1-2C and Comparative Example 1C. The T cells that were not transduced using lentivirus were referred to as a control group (un-transduced group). Herein, the foresaid MOI = [viral titer (TU/mL) $\times$ viral volume (mL)] / total number of T cells. On day 3, following detachment of Dynabeads from the complexes of Dynabeads and CAR-T cells by DynaMag-Spin, CAR-T cells of Examples 1-1C and 1-2C, and Comparative Example 1C were obtained, and the structural schematic illustration of each group is shown in FIG.3. The foresaid resulting CAR-T cells were transferred to a new flask having X-VIVO$^{TM}$ 15 medium containing 200 IU/mL of recombinant human IL-2 (R&D, 202-GMP-01M), and cultured with the same conditions used in the foresaid viral transduction process to make CAR-T cells expand until the 7$^{th}$ day. Last, the cell numbers of CAR-T of the un-transduced group and Examples 1-1C and 1-2C and Comparative Example 1C were measured. During the culture period of CAR-T, additional medium was added every 1 to 3 days for continuously adjusting the cell density to $1 \times 10^6$ cells/mL.

**Test Example 2: Cell expansion experiments of CAR-T cells of Examples 1-1C and 1-2C, and Comparative Example 1C**

The cell expansion rates of CAR-T cells of Examples 1-1C and 1-2C, and Comparative Example 1C were obtained by calculating the total cell number at a specific timepoint during the preparation of CAR-T cells of Examples 1-1C and 1-2C, and Comparative Example 1C. Specifically, on day 3 during the preparation of CAR-T cells, CAR-T cells of Examples 1-1C and 1-2C, and Comparative Example 1C were seeded in 12-well plates at a cell density of $1 \times 10^6$ cells/mL. After Dynabeads were removed, cell viability and cell density were measured by staining cells with Acridine Orange/Propidium Iodide (AO/PI) and then automatically quantifying cell numbers by an automated cell counter (Luna Automated Cell Counter, Logos Biosystems). The remaining cells then were subcultured and seeded in cell culture plates at cell densities between $2 \times 10^5$ cells/mL and $5 \times 10^5$ cells/mL for cell expansion. Cell numbers were measured on day 0 and day 7 of CAR-T cells preparation to calculate for cell expansion rates, and the experimental results were shown in the following Table 2; wherein, the cell expansion rates of CAR-T cells of Examples 1-1C and 1-2C, and Comparative Example 1C were the expansion fold standardized and calculated with the cell number measured on day 0.

Table 2: expansion folds on the 7th day for CAR-T cells of Examples 1-1C and 1-2C, and Comparative Example 1C

| Group | Example 1-1C | Example 1-2C | Comparative Example 1C |
|---|---|---|---|
| Expansion fold on day 7 relative to day 0 | 15.8X | 10.5X | 21.9X |

When the expansion fold can reach greater than 5-fold, it was beneficial to the subsequent functional tests of T cells. Therefore, according to the results in the Table 2, CAR-T cells of Example 1-1C and Example 1-2C, and Comparative Example 1C had good expansion fold for T cells. In Examples 1-1C and 1-2C, CAR-T cells of Example 1-1C having DAP12 intracellular signaling domain as the intracellular signaling domain had better cell expansion effect than CAR-T cells of Example 1-2C having 41BB intracellular signaling domain as the intracellular signaling domain.

**Test Example 3: Expression detection of CAR-T cells of Examples 1-1C and 1-2C, and Comparative Example 1C; transduction efficiencies of lentiviruses of Examples 1-1B and 1-2B, and Comparative Example 1B**

The expressions of NKp30 of CAR-T cells of Examples 1-1C and 1-2C, and Comparative Example 1C were detected by using anti-NKp30 antibody labeled with BV421 fluorescent dye (BioLegend, 325228) and flow cytometer. Then, the transduction efficiencies of lentiviruses of Examples 1-1B and 1-2B, and Comparative Example 1B were the percentage of number of cells that expressed NKp30 relative to total cell number, and the experimental results were shown in the following Table 3.

Table 3: the transduction efficiencies of lentiviruses of Examples 1-1B and 1-2B, and Comparative Example 1B

| Group | Example 1-1B | Example 1-2B | Comparative Example 1B |
|---|---|---|---|
| Transduction efficiency | 96% | 88% | 40% |

When the transduction efficiency of preparing CAR-T cells was greater than 25%, it was beneficial to the subsequent functional tests of expanded CAR-T cells. Therefore, according to the results in Table 3, both the lentiviruses of Examples 1-1B and 1-2B exhibited good transduction efficiency. The transduction efficiency of lentivirus of Example 1-1B having DAP12 as the intracellular signaling domain was better than the lentivirus of Example 1-2B having 41BB as the intracellular signaling domain, and both were better than the lentivirus of Comparative Example 1B.

**Test Example 4: T cell subpopulation analysis for CAR-T cells of Examples 1-1C and 1-2C, and Comparative Example 1C**

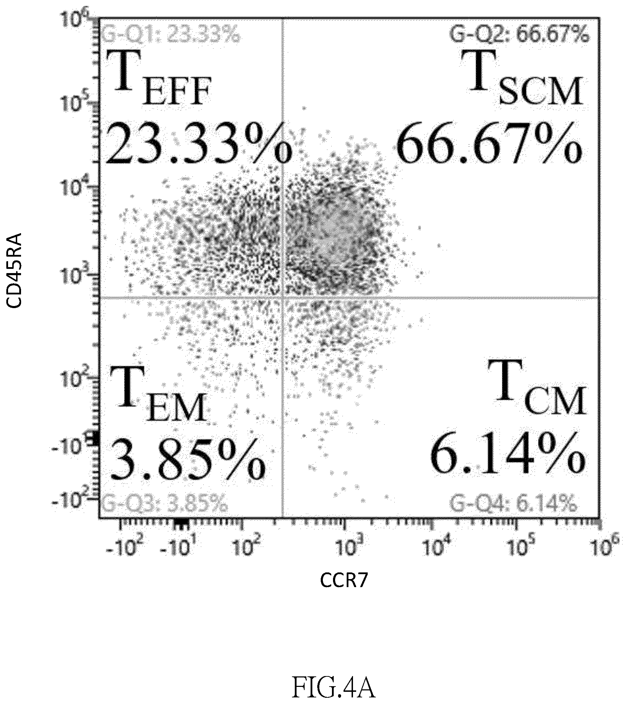

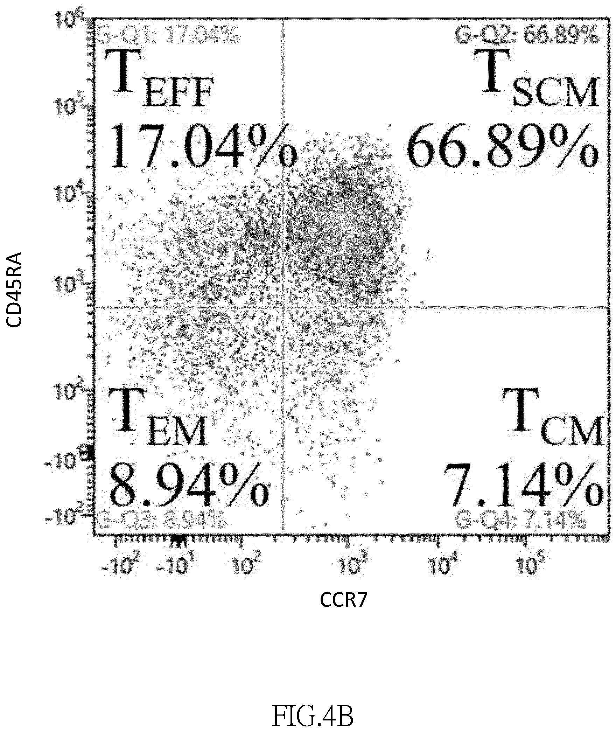

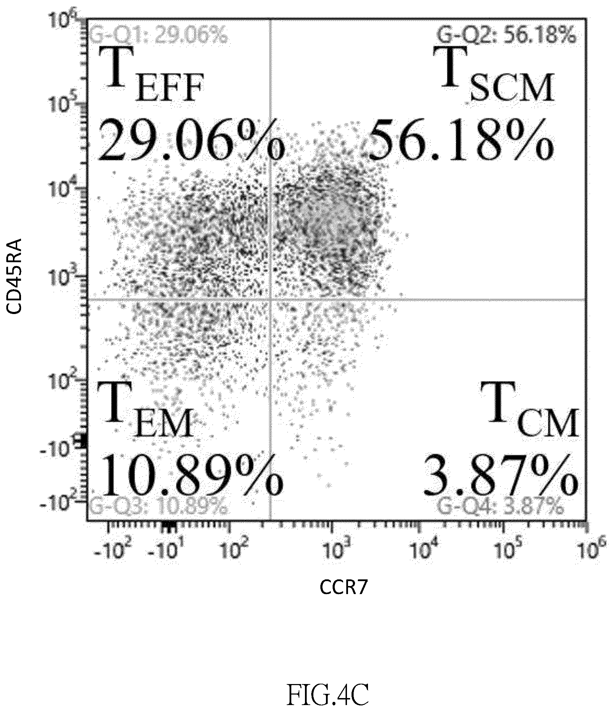

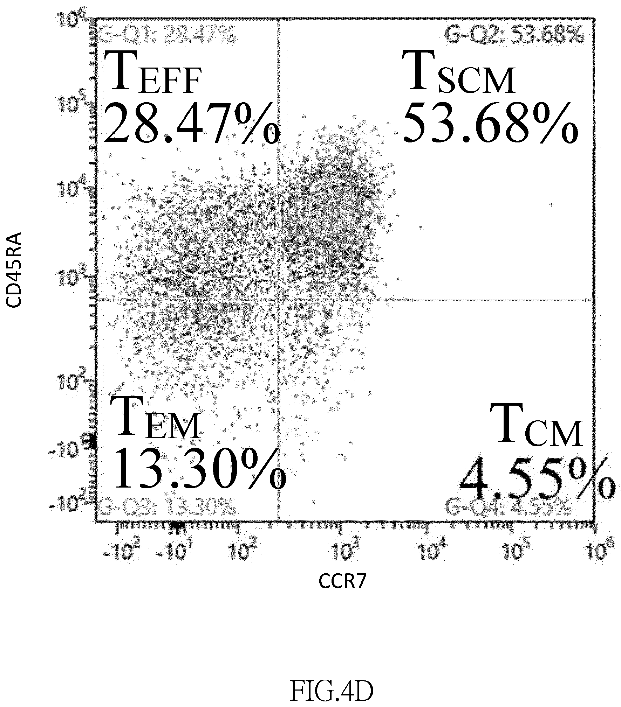

During the preparation of CAR-T cells of Examples 1-1C and 1-2C, and Comparative Example 1C, the T cell subpopulation of CAR-T cells after 7 days of activation were assessed by detecting cell-surface markers of T cells that were fluorescently labeled by antibodies and a flow cytometer with SA3800 software (Sony Biotechnology); wherein, the used antibodies were CD3-FITC (BioLegend, 300406), CD4-PE-Cy7 (BioLegend, 300512), CD8-AF700 (Beckman, B76279), CD95-APC (BioLegend, 305612), CD45RA-PE (BioLegend, 304108) and CCR7-BV421 (BioLegend, 353208). A fluorescence-minus-one (FMO) control group was also included for flow cytometric analysis, which meant that samples were stained with all foresaid fluorescently labeled antibodies except for CCR7-BV421 and CD45RA-PE. In this Test Example, un-transduced, activated T cells were used as a control group (an un-transduced group). The staining results obtained from the foresaid method were used to determine the distribution in percentage of each T cell subpopulation: Tscm, central memory T-cell ($T_{CM}$), effector memory T-cell ($T_{EM}$), or terminally differentiated effector memory T-cell [$T_{EMRA}$; also named as effector T cell ($T_{EFF}$)] in accordance with Annals of Oncology Volume 32, Issue 11, Pages 1366-1380. The percentages of Tscm in CAR-T cells of Examples 1-1C and 1-2C, and Comparative Example 1C are shown in the following Table 4. The results of distribution of the subpopulations of T cells for Examples 1-1C and 1-2C, Comparative Example 1C and the un-transduced group from flow cytometry are shown in FIGs.4A, 4B, 4C and 4D, respectively; wherein, T cells simultaneously expressing CD45RA and CCR7 were considered Tscm, T cells expressing CD45RA but not expressing CCR7 were considered $T_{EMRA}$ (also named as $T_{EFF}$), T cells not expressing CCR7 and CD45RA were considered $T_{EM}$, and T cells expressing CCR7 but not expressing CD45RA were considered $T_{CM}$.

Table 4: the percentages of Tscm for CAR-T cells of Examples 1-1C and 1-2C, Comparative Example 1C, and the un-transduced group

| Group | Example 1-1C | Example 1-2C | Comparative Example 1C | Un-transduced group (control group) |
|---|---|---|---|---|
| Tscm(%) | 66.7% | 66.9% | 56.2% | 53.7% |

If the stem cell-like memory T cell (Tscm) percentage of CAR-T cells is greater than 40%, cytokine release syndrome and neurotoxicity caused by CAR-T cell therapy will be reduced due to high proportion of Tscm. According to the results in Table 4 and FIGs.4A to 4D, both CAR-T cells of Examples 1-1C and 1-2C, which had modified wild-type NKp30 with an intracellular signaling domain, had higher Tscm proportions, and both were higher than CAR-T cells of Comparative Example 1C and the activated T cells of the un-transduced group. Therefore, it can be expected that CAR-T cells of Examples 1-1C and 1-2C were able to reduce cytokine release syndrome and neurotoxicity caused by such therapy.

**Test Example 5: Real-time cytotoxicity assay (RTCA) by using xCELLigence**

Hela cervical cancer cell line (hereinafter referred to as Hela cells) was a mesothelin-positive cell line, and Hela cells (purchased from Elabscience, CL-0101) were cultured in DMEM containing 10% fetal bovine serum (FBS) at 37°C in 5% $CO_2$ for the usage of the subsequent real-time cytotoxicity assay by using xCELLigence.

For measuring the anti-tumor activities of CAR-T cells of Examples 1-1C and 1-2, and Comparative Example 1C, the foresaid HeLa cells were set as target cells for the cytotoxicity assay, and effector cells used in this Test Example were CAR-T cells of Examples 1-1C and 1-2C, and Comparative Example 1C.

The xCELLigence RTCA system (Agilent Technologies, RTCA SP) was placed in a cell incubator at 37°C in 5% $CO_2$, and connected to the interfaces of an external analysis unit and a control unit by cables; meanwhile, the RTCA Software Pro software possessed functions of real-time controlling and monitoring of instruments, including real-time data display and analysis. Specifically, the xCELLigence RTCA system was used to continuously monitor the cytotoxicity effect on tumor cells for 90 hours: first, the foresaid Hela cells were used as target cells (T) and seeded with a cell density of $1.2 \times 10^4$ cells/mL into a culture plate with the background corrected. Then, the background impedance measurement was performed for 22 hours for the attachment of the target cells, and the effector cells (E) were then added into the culture plate for co-culture at an effector cell to target cell ratio (effector-to-target ratio, E:T ratio) of 10:1, 3:1 and 1:1 with the final volume of each well being 200 μL. The effector cells used in this Test Example were CAR-T cells of Examples 1-1C and 1-2, and Comparative Example 1C. In addition, un-transduced, activated T cells were used as a control group (un-transduced group). The cell index (CI) was measured every 15 minutes for at least 72 hours. The raw RTCA impedance background data were normalized with the last measured cell index value before addition of the effector cells. The foresaid cell index was a dimensionless parameter and obtained by measuring the relative change of electrical impedance, which represented the status of cells. For example, when cells did not exist or were unattached on the electrodes, the cell index was zero; and under the same physiological conditions, the more the number of cells attached on the electrodes, the higher the value of cell index. Besides, status changes of cells, such as changes in cell morphology or cell adhesion, could change the cell index.

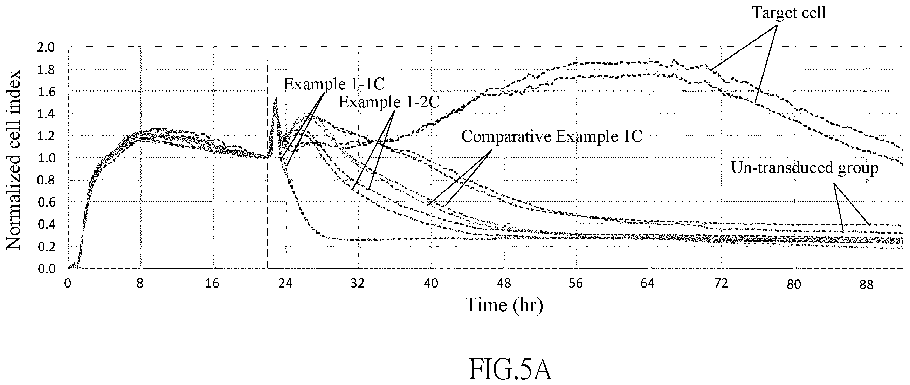

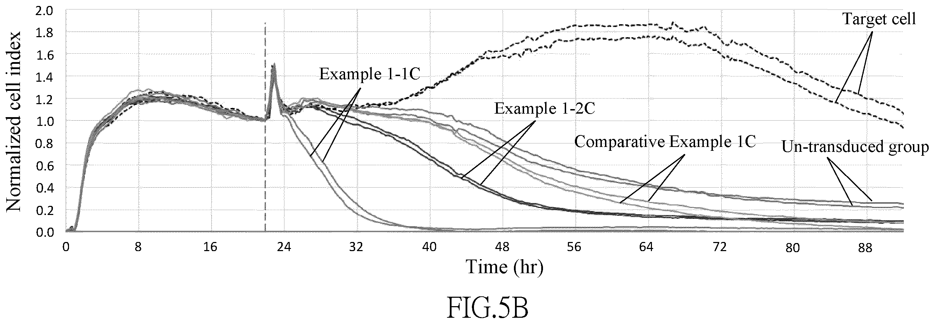

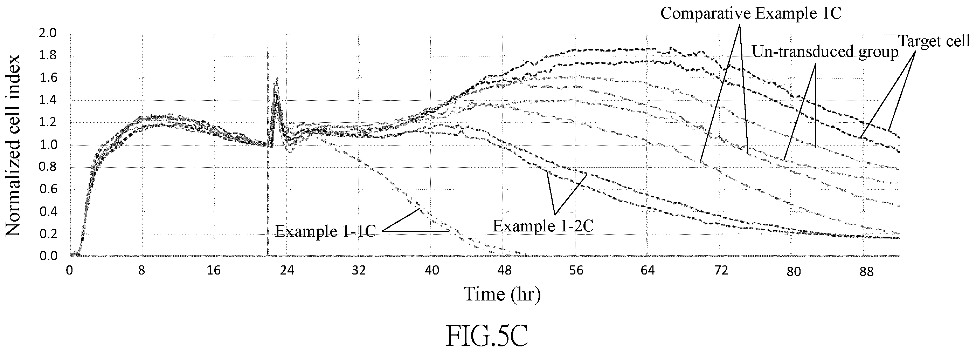

The experimental results are shown in FIGs.5A to 5C, and the results of cytotoxicity for 24 hours were shown in the following Table 5.

Table 5: the results of cytotoxicity assay of CAR-T cells of Examples 1-1C and 1-2C, and Comparative Example 1C

| Group | Example 1-1C | Example 1-2C | Comparative Example 1C |
| --- | --- | --- | --- |
| Specific lysis (E:T=10:1) | 100% | 75% | 50% |

According to the results in Table 5 and FIGs.5A to 5C, both the cytotoxicity of CAR-T cells of Examples 1-1C and 1-2C were better than Comparative Example 1C, and the cytotoxicity at 24 hours of CAR-T cells of Example 1-1C at E:T ratios of 10:1, 3:1 and 1:1 all reached 100%. Because when the cytotoxicity of CAR-T cells exceeds 50%, CAR-T could exhibit sufficient cytotoxicity when it is later changed to a patient's autologous cells for its preparation. According to the results in above Table 5, both CAR-T cells of Examples 1-1C and 1-2C demonstrated sufficient cytotoxicity effect, and the cytotoxicity of CAR-T of Example 1-1C transduced with DAP12 intracellular signaling domain was better than the cytotoxicity of CAR-T cells of Example 1-2C transduced with 41BB intracellular signaling domain.

In summary, the lentiviruses of Examples 1-1B and 1-2B of the present invention have intracellular signaling domain and NKp30 complete fragment with NKp30 transmembrane domain of Comparative Example and Example, thereby both had good transduction efficiencies; and the produced CAR-T cells after transduction had superior CAR-T cell expansion rate and excellent cytotoxicity, and higher proportions of Tscm, which could be expected to reduce cytokine release syndrome and neurotoxicity caused by CAR-T cell therapy. Therefore, in the preparation of CAR-T cells, if a nucleic acid sequence of intracellular signaling domain, such as DAP12 or 41BB intracellular signaling domain, is transduced, they can bring excellent cytotoxicity effect for the produced CAR-T cells, and is especially suitable for CAR-T cell therapy.

**II. Preparation and test of CAR-T cells of anti-mesothelin-NKp30 which has an intracellular signaling domain**

**Example 2: Nucleic acid molecule having intracellular signaling domain, scFv of anti-mesothelin antibody and NKp30 fragment without immunoglobulin-like domain**

The 3' end of a nucleic acid sequence of SS1 scFv of anti-mesothelin antibody (shown as SEQ ID NO: 85) was linked to the 5' end of a nucleic acid sequence of NKp30 fragment without immunoglobulin-like domain (shown as SEQ ID NO: 86) by a nucleic acid sequence of hinge domain of $(G_4S)_3$ (shown as SEQ ID NO: 66). Wherein the nucleic acid sequence of NKp30 fragment without immunoglobulin-like domain comprised extracellular stalk domain, NKp30 transmembrane domain and NKp30 cytoplasmic domain, but did not comprise an immunoglobulin-like domain. Then, the 3' end of the nucleic acid sequence of NKp30 fragment without immunoglobulin-like domain was linked to the 5' end of a nucleic acid sequence of DAP12 intracellular signaling domain (shown as SEQ ID NO: 78), to obtain a linked nucleic acid molecule fragment 2. Then, the 5' end of the linked nucleic acid molecule fragment 2 was linked to the 3' end of a nucleic acid sequence of full length $Fc\varepsilon R1^{\gamma}$ (shown as SEQ ID NO: 77) by a nucleic acid sequence of T2A self-cleaving peptide (shown as SEQ ID NO: 80), to obtain a nucleic acid molecule having intracellular signaling domain, scFv of anti-mesothelin antibody and NKp30 fragment without immunoglobulin-like domain of Example 2, which was shown as SEQ ID NO: 87.

The nucleic acid molecule of Example 2 was, from the 5' end to the 3' end, the nucleic acid sequence of full length $Fc\varepsilon R1^{\gamma}$, the nucleic acid sequence of T2A self-cleaving peptide, the nucleic acid sequence of SS1, the nucleic acid sequence of the hinge domain of $(G_4S)_3$, the nucleic acid sequence of NKp30 fragment without immunoglobulin-like domain and the nucleic acid sequence of DAP12 intracellular signaling domain.

**Comparative Example 2: Nucleic acid molecule having intracellular signaling domain and scFv of anti-mesothelin antibody, without any NKp30 fragment**

The 3' end of the nucleic acid sequence of SS1 scFv of anti-mesothelin antibody (shown as SEQ ID NO: 85) was linked to the 5' end of a nucleic acid sequence of CD8 transmembrane domain (shown as SEQ ID NO: 88) by a nucleic acid sequence of CD8 hinge domain (shown as SEQ ID NO: 67). Then, the 3' end of the nucleic acid sequence of CD8 transmembrane domain was further linked to the 5' end of a nucleic acid sequence of 4-1BB intracellular signaling domain (shown as SEQ ID NO: 82). Then, the 3' end of the nucleic acid sequence of 4-1BB intracellular signaling domain was linked to the 5' end of a nucleic acid sequence of signal transduction domain of CD3ζ (shown as SEQ ID NO: 89), to obtain a nucleic acid molecule of Comparative Example 2 (shown as SEQ ID NO: 90). The nucleic acid molecule of Comparative Example 2 was, from the 5' end to the 3' end, the nucleic acid sequences encoding SS1, the hinge domain of CD8, the transmembrane domain of CD8, the intracellular signaling domain of 4-1BB and the signal transduction domain of CD3ζ.

**Example 2A and Comparative Example 2A: Construction of plasmids with the nucleic acid molecules of Example 2 or Comparative Example 2**

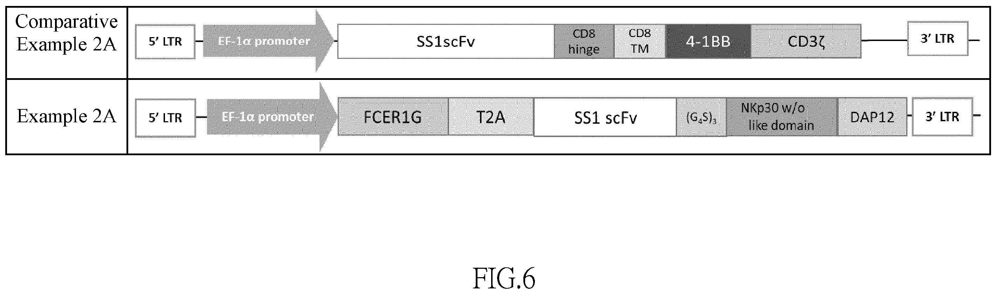

The preparation method of Example 2A was similar to the preparation methods of Examples 1-1A and 1-2A, except that the intracellular signaling domain, scFv of anti-mesothelin antibody and NKp30 fragment without immunoglobulin-like domain of Example 2 were ligated into the foresaid pLAS5w vector by restriction enzymes in Example 2A. While, in Comparative Example 2A, the nucleic acid molecule of Comparative Example 2 was ligated into pLAS5w.Ppuro vector (purchased from Academia Sinica RNAi Core facility, Product No.: C6-8-39) by restriction enzymes of HpaI and EcoRI (conducted by GenScript). The prepared plasmid of anti-mesothelin-NKp30 of Example 2A which has an intracellular signaling domain and the plasmid of Comparative Example 2A are shown in FIG.6.

**Example 2B and Comparative Example 2B: Preparation of lentiviruses with the nucleic acid molecules of Example 2 and Comparative Example 2**

The preparation method of Example 2B was similar to the preparation methods of Examples 1-1B and 1-2B, and the difference was that the plasmid of Example 2A was adopted as the transfer plasmid in Example 2B. While, the plasmid of Comparative Example 2A was adopted as the transfer plasmid for the lentivirus of Comparative Example 2B.

**Test Example 6: Titer determination of lentiviruses of Example 2B and Comparative Example 2B**

The method in Test Example 6 was similar to Test Example 1, and the difference was that Biotinylated Human Mesothelin, His, Avitag (MSN-H82E9, ACROBiosystems) was adopted for targeting anti-mesothelin receptor and 500-fold diluted Streptavidin conjugated phycoerythrin (PE) (Invitrogen) was adopted for detecting the expression of anti-mesothelin receptor of lentiviruses of Example 2B and Comparative Example 2B. The test results are shown in the following Table 6.

Table 6: the functional viral titers of lentiviruses of Example 2B and Comparative Example 2B

| Group | Example 2B | Comparative Example 2B |
|---|---|---|
| Functional viral titer (TU/mL) | $2\times10^7$ | $3.9\times10^7$ |

When the functional viral titer was more than or equal to $1\times10^6$ TU/mL, when preparing CRT-T cells, T cells could be successfully transduced and become CAR-T cells with a lower amount of lentivirus, and the transduced T cells tended to have better growth. Therefore, according to the results in above Table 6, Example 2B and Comparative Example 2B met the foresaid criteria, which further benefited the subsequent preparation of CAR-T cells.

**Example 2C: CAR-T cells of anti-mesothelin-NKp30 which has an intracellular signaling domain**

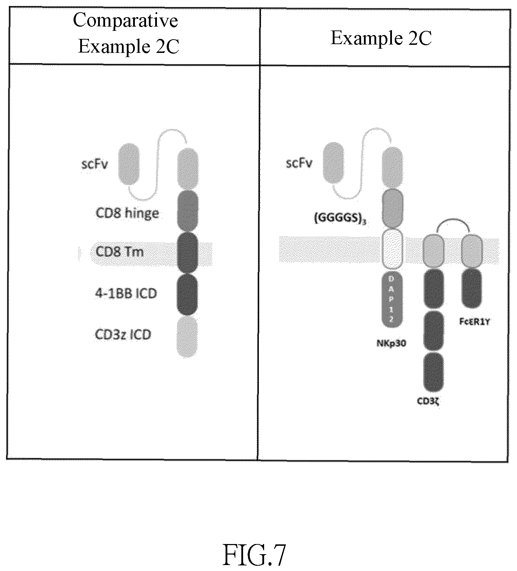

The preparation method of CAR-T cells of anti-mesothelin-NKp30 which has an intracellular signaling domain of Example 2C was similar to the preparation methods of CAR-T cells of Examples 1-1C and 1-2C, and the difference was that the T cells were transduced with the lentivirus of Example 2B and the MOI of lentivirus transduction was 2 in this Example. While, CAR-T cells of Comparative Example 2C were prepared by transducing with the lentivirus of Comparative Example 2B and the MOI of lentivirus transduction was 2. The structural schematic illustrations of the prepared CAR-T cells of Example 2C and CAR-T cells of Comparative Example 2C are shown in FIG.7; wherein, CD3ζ of Example 2C was inherent in T cells; wherein, because the nucleic acid sequences transduced into CAR-T cells of Example 2C and Comparative Example 2C had FcεR1γ, the resulting CAR-T cells were multi-chain CAR-T cells.

**Test Example 7: Cell expansion experiments of CAR-T cells of Example 2C and Comparative Example 2C, and T cells of the un-transduced group**

The method in Test Example 6 was similar to Test Example 2, and the difference was that the cell expansion rate of CAR-T cells of Example 2C was detected in this Test Example, and the un-transduced, activated T cells were set as a control group. The results are shown in the following Table 7.

Table 7: Expansion folds on day 7 of CAR-T cells of Example 2C and Comparative Example 2C, and T cells of the un-transduced group

| Group | Example 2C | Comparative Example 2C | Un-transduced group (control group) |
|---|---|---|---|
| Expansion folds on day 7 relative to day 0 | 29.4X | 18.87X | 29.5X |

When the expansion rate was greater than 5-fold, it was beneficial to the subsequent functional tests of T cells. Therefore, according to the results in above Table 7, CAR-T cells of Example 2C had superior expansion fold for T cells, which appeared to be superior than that of CAR-T cells of Comparative Example 2C.

**Test Example 8: Expression detection of CAR-T cells of Example 2C and Comparative Example 2C; transduction efficiencies of lentiviruses of Example 2B and Comparative Example 2B**

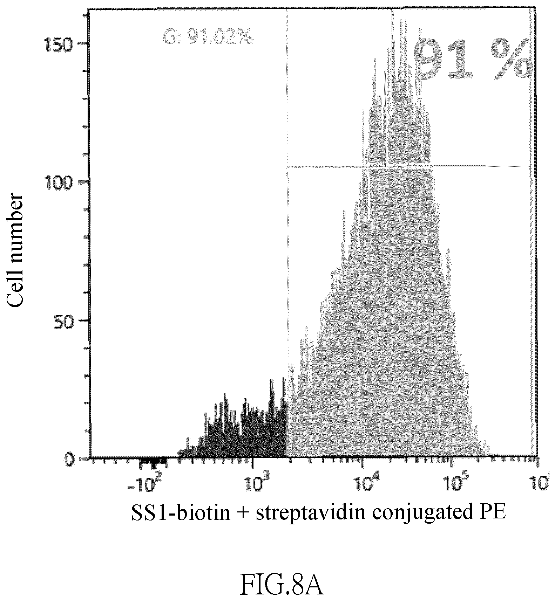

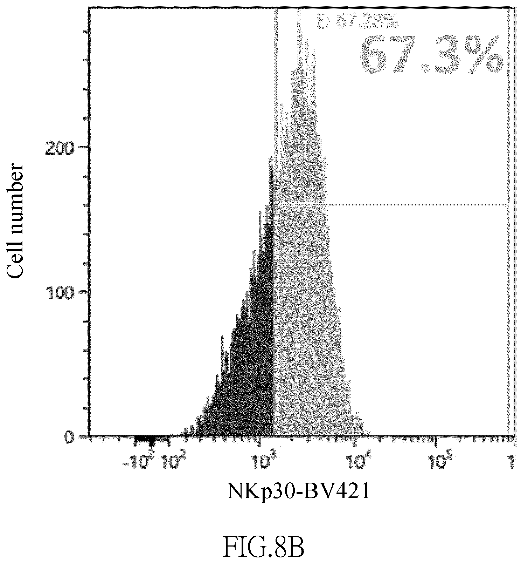

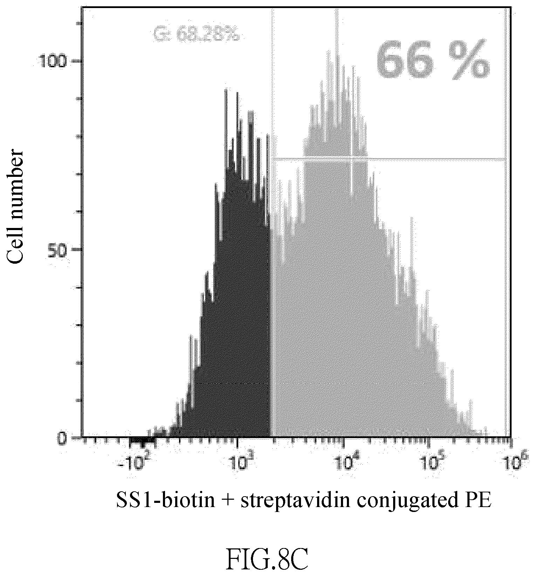

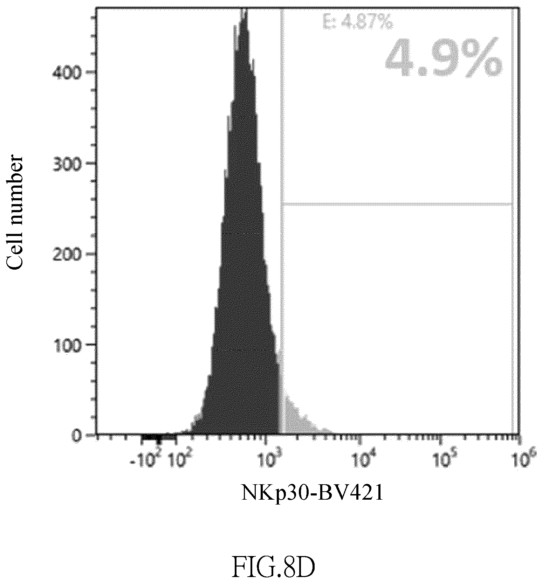

The method in Test Example 8 was similar to that of Test Example 3, and the difference was that Biotinylated Human Mesothelin, His, Avitag (MSN-H82E9, ACROBiosystems) was used to target anti-mesothelin receptor and 500-fold diluted Streptavidin conjugated PE (Invitrogen) was used to detect the expressions of anti-mesothelin receptor of CAR-T cells of Example 2C and Comparative Example 2C. The test results of Example 2C and Comparative Example 2C were respectively shown as the flow cytometry results in FIGs.8A, 8B, 8C and 8D. Then, the transduction efficiencies of lentiviruses of Example 2B or Comparative Example 2B were the percentage of the number of cells expressing the anti-mesothelin receptor relative to the total cell number, and the experimental results were shown in the following Table 8.

Table 8: the transduction efficiencies of lentiviruses of Example 2B and Comparative Example 2B

| Group | Example 2B | Comparative Example 2B |
|---|---|---|
| Transduction efficiency | 70% to 100% | 60% to 80% |

When the transduction efficiency of preparing CAR-T cells was greater than 25%, it was beneficial to the subsequent functional tests of expanded CAR-T cells. Therefore, according to the results in above Table 9 and FIG.8A, the lentivirus of Example 2B exhibited better transduction efficiency compared to Comparative Example 2B.

**Test Example 9: T cell subpopulation analysis for CAR-T cells of Example 2C and Comparative Example 2C, and T cells of the un-transduced group**

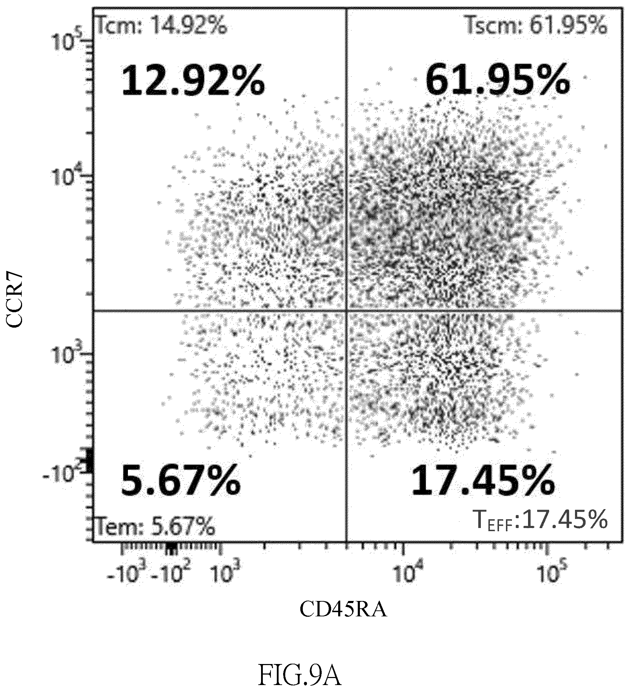

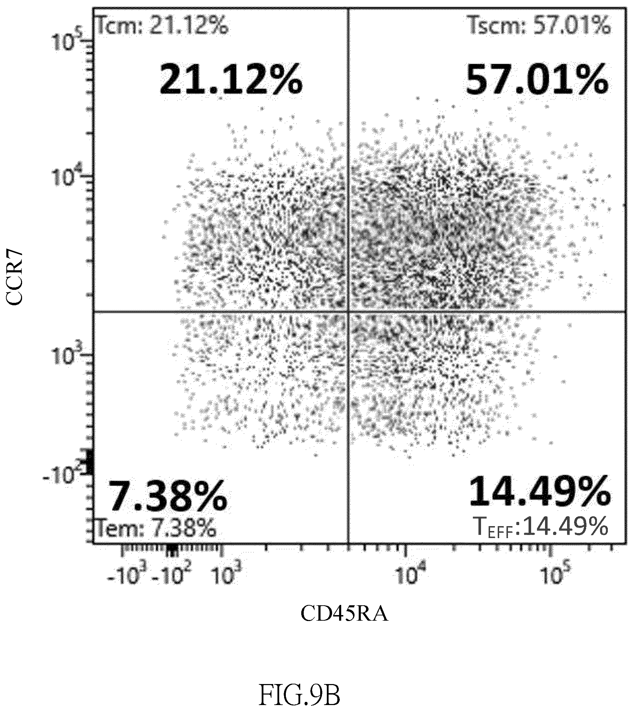

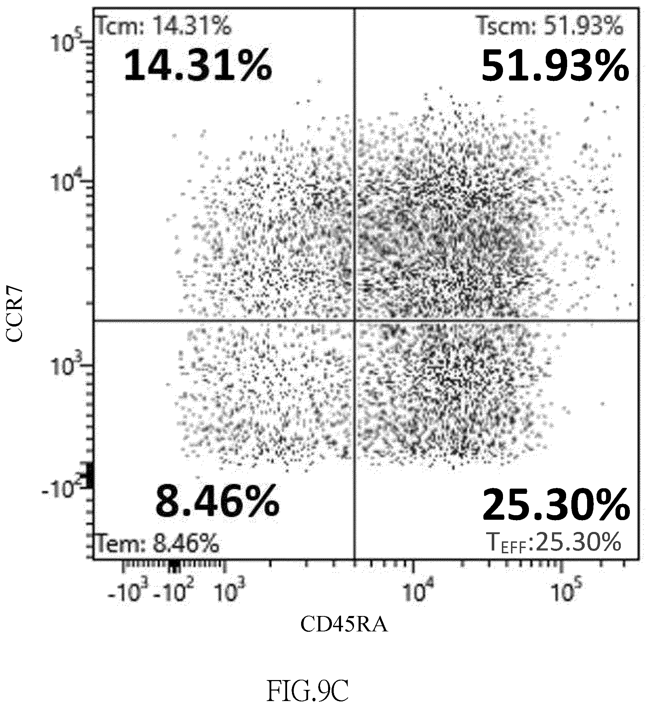

The method in Test Example 9 was similar to Test Example 4, and the difference was that the analyses in this Test Example were performed on the 7th day of the preparation of CAR-T cells of Example 2C. The percentages of Tscm in CAR-T cells of Example 2C and Comparative Example 2C, and in T cells of the un-transduced group were shown in the following Table 9; and the results of the distribution of the subpopulations of T cells of Example 2C result, Comparative Example 2C and the un-transduced group from flow cytometry were shown in FIGs.9A, 9B and 9C, respectively; wherein, T cells simultaneously expressing CD45RA and CCR7 were considered Tscm.

Table 9: the percentages of Tscm in CAR-T cells of Example 2C and Comparative Example 2C, and T cells of the un-transduced group

| Group | Example 2C | Comparative Example 2C | Un-transduced group |
|---|---|---|---|
| Tscm(%) | 62% | 57% | 52% |

If the percentage of Tscm in CAR-T cells is greater than 40%, cytokine release syndrome and neurotoxicity caused by CAR-T cell therapy can be reduced. According to above Table 9 and FIGs.9A, 9B and 9C, Example 2C, which had intracellular signaling domain, displayed higher proportions of Tscm, was better than CAR-T cells of Comparative Example 2C and activated T cells of the un-transduced group. Therefore, it can be expected that CAR-T cells of Example 2C were able to reduce cytokine release syndrome and neurotoxicity caused by such therapy.

**Test Example 10: Real-time cytotoxicity assay by using xCELLigence**

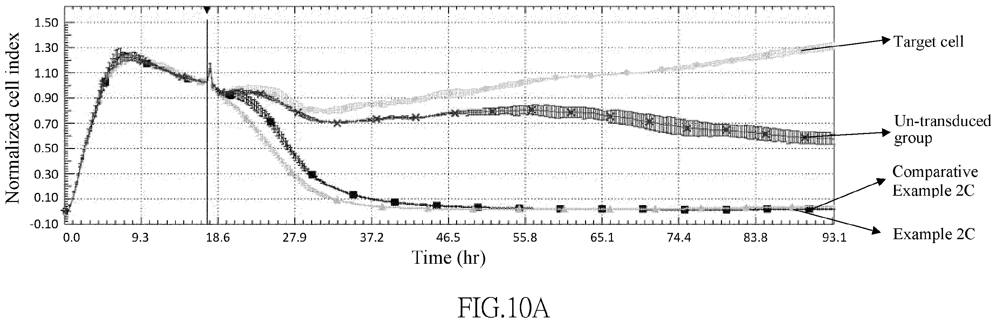

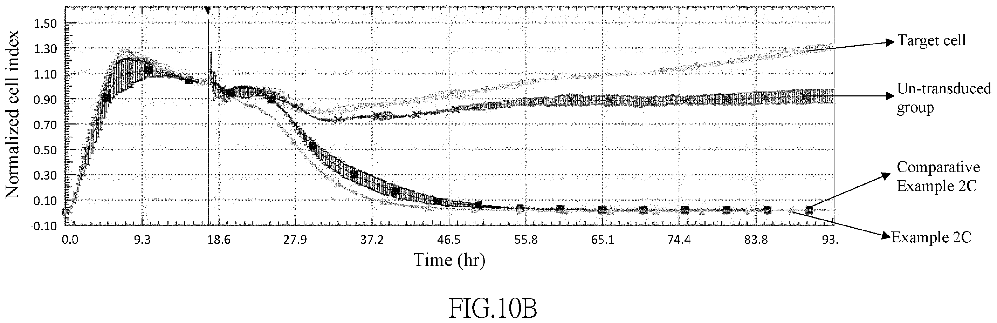

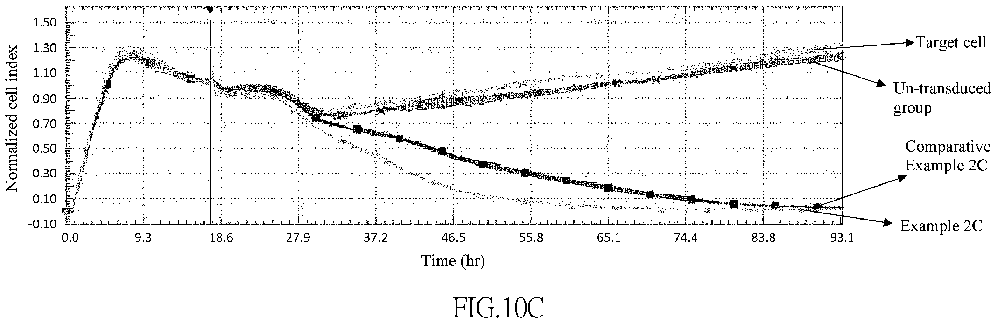

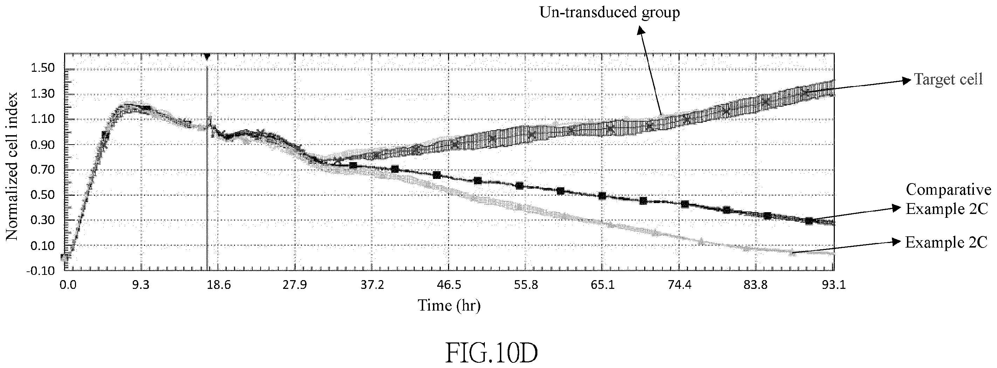

The method in Test Example 10 was similar to Test Example 5, and the difference was that the effector cells were CAR-T cells of Example 2C or Comparative Example 2C in this Test Example, and the target cells were the foresaid Hela cells. The target cells were seeded in a culture plate (E-plate 96 well, Agilent Technologies, 300600910) with the background corrected for overnight culture to let the target cells attach and then proceed 18 hours of impedance measurement. Then, the effector cells were added into the culture plate at E:T ratios of 3:1, 1:1, 0.3:1 and 0.1:1 for test. The experimental results of cytotoxicity are shown in FIGs.10A, 10B, 10C and 10D, respectively.
And, the results of cytotoxicity for 24 hours were shown in the following Table 10.

Table 10: the results of real-time cytotoxicity assay of CAR-T cells of Example 2C and Comparative Example 2C

| Group | Example 2C | Comparative Example 2C |
|---|---|---|
| Specific lysis (E:T=1:1) | 65% | >60% |

According to the results in Table 10 and FIGs.10A to 10D, the cytotoxicity of CAR-T cells of Example 2C was better than traditional single-chain CAR-T cells of Comparative Example 2C. Specifically, at E:T ratios of 0.3:1 and 0.1:1, the foresaid cytotoxicity effect of Example 2C was obviously more significant than Comparative Example 2C, and the standardized cell index value was significantly decreased, which represented that numerous target cells were killed through cytotoxicity. Besides, when the cytotoxicity of CAR-T cells exceeds 50%, CAR-T could exhibit sufficient cytotoxicity when it is later changed to a patient's autologous cells for its preparation. According to the results in above Table 10, CAR-T cells of Example 2C demonstrated excellent cytotoxicity effect.

**Test Example 11: Luciferase killing assay**

SKOV3 ovarian cancer cells (hereinafter referred to as SKOV3 cells; purchased from ATCC, Cat. No. HTB-77) were cultured in McCoy 5A Medium (Gibco, 16600082) containing 10% FBS. Then, anti-mesothelin antibody labeled with green fluorescent protein and a flow cytometry cell sorter were used to screen out SKOV3 cells expressing high level of mesothelin. To establish a luciferase-expressing SKOV3 cell line, lentivirus vector PLL-CMV-rFluc-T2A-GFP-mPGK Lenti-Labeler (System Biosciences) was transduced into the foresaid SKOV3 cells expressing high level of mesothelin. Then, single cell lines capable of stable expression were screened out to obtain GFP/Luc$^+$ SKOV3 cells (SKOV3-mLE) that could simultaneously express green fluorescent protein (GFP) and a firefly luciferase enzyme.

In order to determine the anti-tumor activities of CAR-T cells of Example 2C and Comparative Example 2C, and activated T cells of the un-transduced group, the cells prepared on day 7 were adopted. The foresaid SKOV3 cells expressing mesothelin and luciferase enzyme were adopted as the target cells to conduct the cytotoxicity assay. The effector cells used in this Test Example were CAR-T cells of Example 2C, and un-transduced T cells were used as a control group (un-transduced group). Specifically, the effector cells and the target cells were co-cultured in 96-well microplates at 37°C in 5% $CO_2$ for 48 hours with an effector-to-target ratio (E:T ratio) of 3:1, and with the experiment performed in triplicates.

Luciferase Assay System (Promega, E1501) was used to quantify firefly luciferase activity, displayed by viable luciferase-expressing SKOV3 cells, by providing luciferin for bioluminescent reaction. The relative numbers of surviving cancer cells, not lysed by CAR-T cells, can be evaluated based on luciferase detection. Consequently, the results of specific cell lysis ratios were calculated using the following equation:

specific cell lysis ratio (%) = 100% $\times$ (number of lysed target cells due to treatment) - (number of spontaneously lysed target cells) / [(maximum number of lysed target cells) - (number of spontaneously lysed target cells)];

wherein, "the number of lysed target cells due to treatment" = the number of lysed target cells obtained from the group of co-culturing the target cells with the effector CAR-T cells;
the "number of spontaneously lysed target cells" = the number of lysed target cells obtained from the group in the absence of the effector cells, *i.e.*, the number of lysed cells after culturing the target cells in RPMI-1640 medium containing 10% FBS at 37°C in 5% $CO_2$ for 48 hours;
the maximum number of lysed target cells = the number of lysed target cells of the group in the absence of the effector cells after the addition of 100 $\mu$L of 1% Triton X-100. The experimental results are shown in the following Table 11.

Table 11: the results of luciferase killing assay (specific cell lysis ratio %) of CAR-T cells of Example 2C and Comparative Example 2C, and T cells of the un-transduced group (control group)

| Group | Example 2C | Comparative Example 2C | Un-transduced group (control group) |
|---|---|---|---|
| Specific lysis (at E:T=3:1) | 90% | 60% | about 0% |

When the cytotoxicity of CAR-T cells exceeds 50%, CAR-T produced using patient's cells should also exhibit sufficient cytotoxicity. According to the results in Table 11, after co-culturing with the target cells for 48 hours, the cytotoxicity effect of CAR-T cells of Example 2C was obviously better than traditional single-chain CAR-T cells of Comparative Example 2C. Therefore, it could be speculated that CAR-T cells of Example 2C had sufficient cytotoxicity when it is later changed to patient's cells for CAR-T preparation.

**Test Example 12: Animal experiment**

Forty 5-10 week-old NOD.Cg-Prkdc$^{scid}$Il2rg$^{tm1Wjl}$B2$^{mem1Narl}$/YckNarl B2m gene deletion immunodeficient (abbreviated as ASID-B2m KO) female mice were provided. The mice were divided into 8 groups (including a backup group which served as a positive control for the tumor re-challenge assay) with 5 mice in each group, and bred in a sterile environment according to the regulations of Institutional Animal Care and Use Committee (IACUC). Because CAR-T cells of the present invention was derived from human T cells, it was expected that the mouse would experience graft-versus-host disease (GvHD) when xenogeneic human T cells were implanted into the immunodeficient experimental mouse. However, based on prior experiments, GvHD associated symptoms could be substantially alleviated with the deletion of the beta-2-microglobulin (B2m) gene. Therefore, immunodeficient mouse with B2m gene deletion were adopted for this Test Example.

$5\times10^6$ cells of the SKOV3-mLE cells derived from Test Example 11 were suspended in 100 $\mu$L of PBS, and then mixed with equal volume of Matrigel (BD Bioscience) to obtain an injection mixture of SKOV3-mLE cells and Matrigel. On day 0, the injection mixture was subcutaneously inoculated to the right flank of 35 mice, excluding the 5 mice. In vivo imaging system (IVIS) (Perkin Elmer) was used to measure the signals of bioluminescence imaging (BLI) weekly, starting from day 10 and until day 80. However, for mice that were sacrificed in advance due to meeting the humane sacrifice criteria, the measurements will not last to day 80. On day 21 following SKOV3-mLE cells (target cells, "T") implantation, aside from mice not inoculated with the SKOV3-mLE cells, mice from the other 7 groups were injected with saline (control group), or CAR-T cells of Example 2C, or CAR-T cells of Comparative Example 2C or the un-transduced T cells (effector cells, "E") via the caudal artery; wherein, the E:T ratio was 1:1 or 3:1. On day 55, tumor re-challenge assay was initiated by injecting a mixture of SKOV3-mLE cells and Matrigel in the contralateral flank, *i.e.*, left side, of remaining live mice and of untreated mice in the backup group. For the re-challenge assay, a total of 22 mice were injected with the SKOV3-mLE cells, wherein 5 backup mice were given cancer cells for the first time. In this Test Example, the logarithmic scales of quantified BLI were used to represent the *in vivo* tumor volume of mice. T cells in the peripheral blood of mice were also collected for analysis of CAR expression and T cell subpopulations; wherein the analysis of CAR expression was described in Test Example 8, and the analysis of T cell subpopulations was described in Test Example 9. The experimental data were expressed by mean ± standard deviation, and statistical analyses were performed using one-way analysis of variance followed by post hoc Tukey tests.

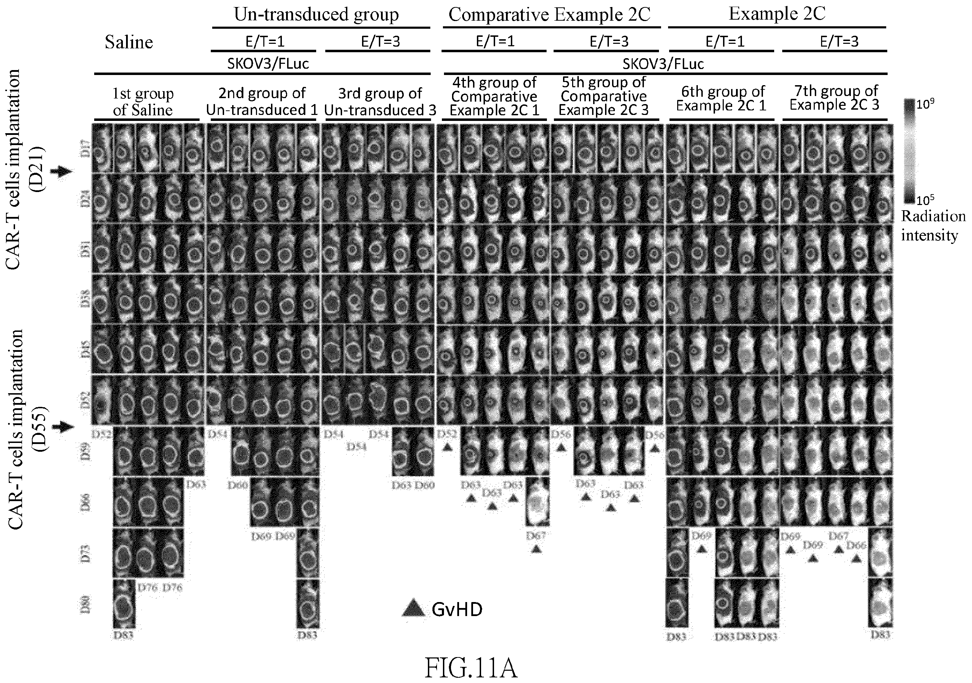

The experimental results were shown in the following Tables 12 and 13, and in FIGs 11A, 11B, 12A, 12B, 13A and 13B. In Table 12, "day -4" was 4 days before administering the effector cells, and "day +10", "day +17", "day +24" and "day +31" were 10 days, 17 days, 24 days and 31 days after administering the effector cells, respectively.

Table 12: the results of the *in vivo* cytotoxicity assay [log(*E+11) photon/second] of Example 2C (abbreviated as E2C) and Comparative Example 2C (abbreviated as CE2C), evaluated by BLI intensities of animal tumors

| Groups \ Days | Un-transduced group | Data comparison between un-transduced and CE2C | Data comparison between un-transduced and E2C | CE2C (E:T=3:1) | Data comparison between CE2C and E2C | E2C (E:T=3:1) |
|---|---|---|---|---|---|---|
| day -4 | 11.0±0.4 | ns | ns | 11.1±0.3 | ns | 11.1±0.3 |
| day +10 | 11.1±0.1 | ns | ** | 10.6±0.2 | * | 9.8±0.8 |
| day +17 | 11.6±0.3 | ** | *** | 10.3±0.3 | *** | 7.8±0.7 |
| day +24 | 11.7±0.2 | *** | *** | 9.9±0.8 | *** | 7.2±0.3 |
| day +31 | 11.9±0.2 | ** | *** | 9.1±1.6 | * | 7.0±0.6 |

ns represented not statistically significant; * presented $p<0.05$, ** presented $p<0.01$ and *** presented $p<0.005$

Table 13: the *in vitro* test data for properties and functions of the effector cells after the effector cells of CAR-T were prepared and before being administered to mice (Comparative Example 2C and Example 2C were abbreviated as CE2C and E2C, respectively)

| Groups | Un-transduced group | Un-transduced group vs CE2C | Un-transduced group vs E2C | CE2C (E:T=3:1) | CE2C vs E2C | E2C (E:T=3:1) |
|---|---|---|---|---|---|---|
| CAR expression % | ~0.0±2.3 | *** | *** | 70.1±2.3 | * | 90.6±5.8 |
| CD8+ T cells (%) | 61.2±4.5 | ns | ns | 56.0±12.5 | ns | 56.7±6.4 |
| Tscm proportion | 50.6±2.8 | ns | ns | 54.8±13.2 | ns | 49.5±2.0 |

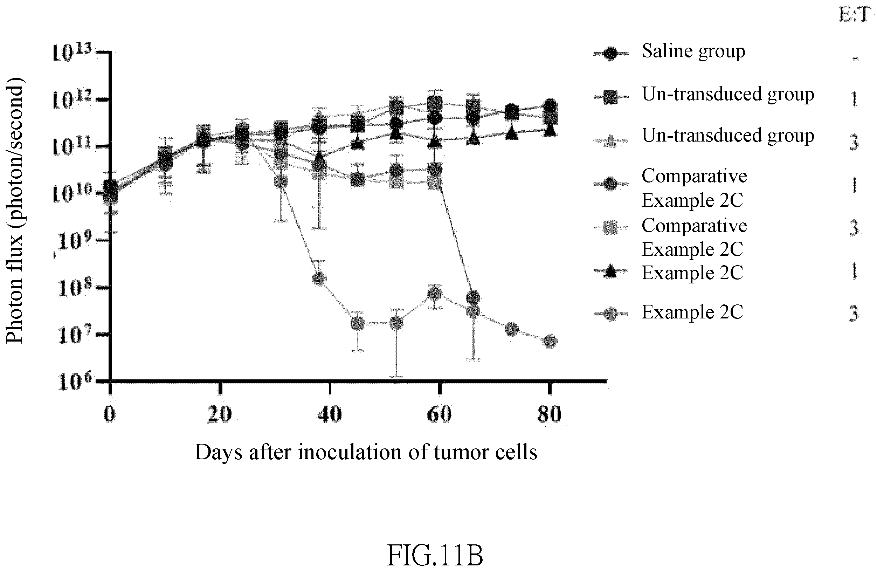

As shown in FIG.11A, with higher injection dose (E:T=3:1), CAR-T cells of Example 2C could reduce tumor volume after 10 days (on day 31 of entire experimental period). According to visual observation for BLI, in mice given high dose of CAR-T cells of Example 2C, the BLI areas of 3/5 mice therein could be detected and the detectable BLI areas showed smaller area compared to other groups of different effector cells. On average, the BLI signals of the group given high dose of CAR-T cells of Example 2C, after transforming to logarithmic scales, was also significantly lower than all other groups (as shown in Table 12 and FIG.11B).

As shown in FIG.11A, after treating with CAR-T cells for 24 days (day 45 overall), in mice given high dose of CAR-T cells of Example 2C, the BLI signals could no longer be observed visually ($<10^8$ photon/second). In mice treated with two doses of CAR-T cells of Comparative Example 2C or low doses (E:T=1:1) of CAR-T cells of Example 2C, obvious decrease in the detectable BLI areas could also be observed; however in these mice treated with different groups, the BLI signals thereof did not reach the point that could not be visually detected. In mice treated with CAR-T cells of Example 2C and Comparative Example 2C, the BLI signals thereof were both lower than their individual UTD control group; and in the mice treated with high dose of CAR-T cells of Example 2C, the BLI value thereof was significantly lower than other groups, including other groups subjected to CAR-T cells treatments, *i.e.*, CAR-T cells of Comparative Example 2C and low dose of CAR-T cells of Example 2C. The differences among these detectable BLI areas continued to day 31 after treatment (day 52 overall), *i.e.*, before the life-threatening GvHD occurred in some mice (as shown in FIGs 11A, 11B and Table 12).

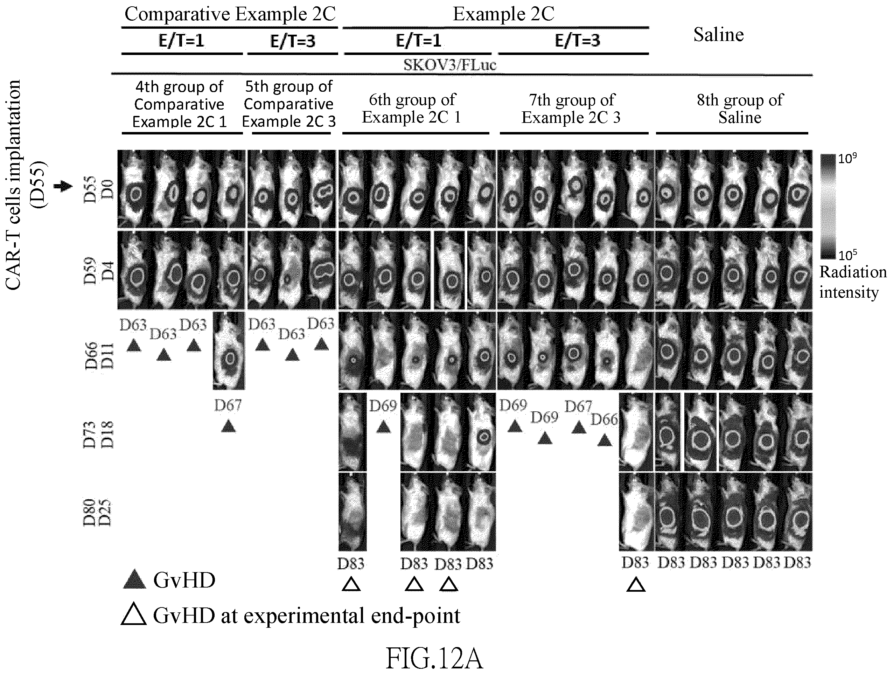

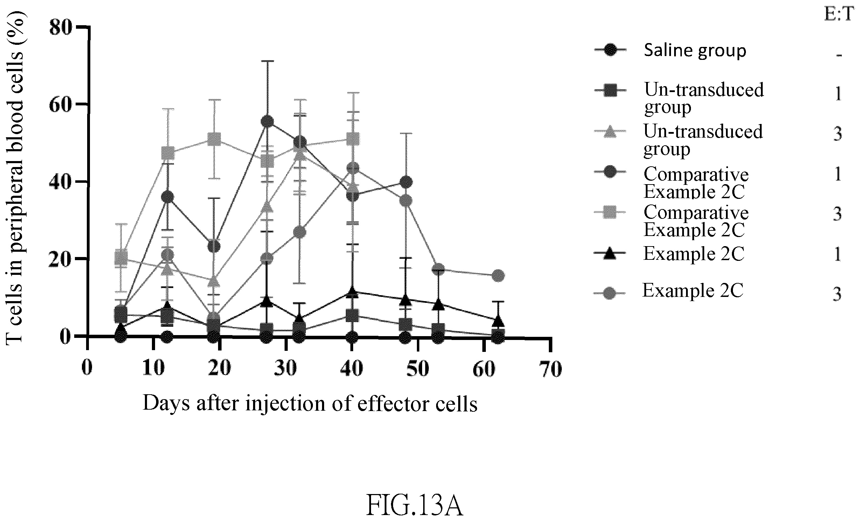

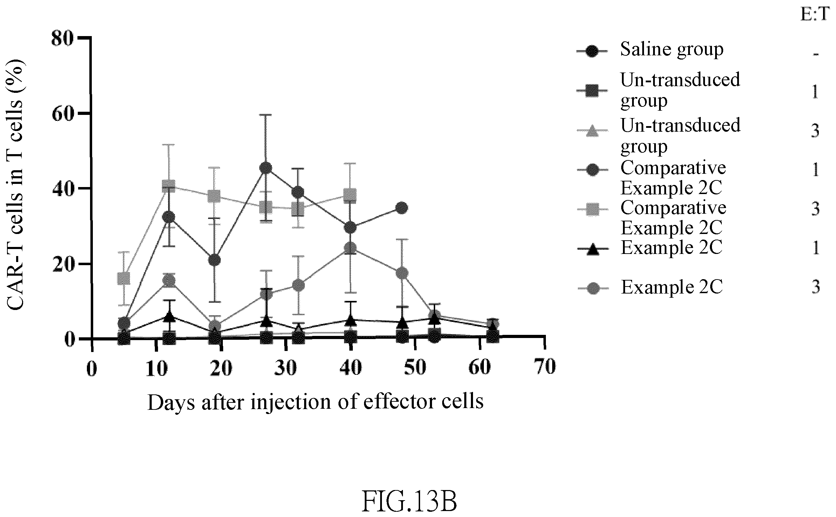

In addition, according to FIG.11A, tumor elimination (decrease in BLI signal intensity) could be observed in FIG.11A, without immediately causing GvHD in mice from the Example 2C treatment group, wherein traditional single-chain CAR-T cells from Comparative Example 2C immediately cause GvHD. Although by using the ASID-B2m KO mice, the symptom of GvHD had been obviously alleviated or delayed, the sign of GvHD was still obvious for mice treated with traditional single-chain CAR-T cells (Comparative Example 2C). The GvHD may result from over-proliferation of human CAR-T cells and the phenomenon of attacking host cells due to the absence of human cancer cells as target cells. Before conducting re-challenge assay by using the SKOV3-mLE cancer cells, 3 mice treated with CAR-T cells of Comparative Example 2C group (accounting for 3/10 of the total number of Comparative Example 2C group) were sacrificed because of GvHD (as shown in FIG.11A). Eleven days into the tumor re-challenge assay, most live mice treated with CAR-T cells of Comparative Example 2C (6 mice in 7 mice) were sacrificed because of GvHD and before the efficacy of CAR-T could be evaluated (as shown in FIG.12A). According to FIGs.13A and 13B, during the entire experimental processes, the total number of T cells (identified as CD3$^+$ cells) in peripheral blood and the percentage of CAR-T cells in T cells of the mice treated with CAR-T cells of Comparative Example 2C were significantly higher than that of Example 2C, which might indicate that the design of traditional single-chain CAR-T cells might tend to produce hyperactive CAR-T cells. According to Table 13, for CAR-T cells of Example 2C, the Tscm proportions were not worse than the un-transduced group, and it was expected to reduce cytokine release syndrome and neurotoxicity induced by CAR-T cell therapy; and according to the descriptions in literature: Galli E. et al. Br J Haematol. 2023 Nov;203(4):564-570, CAR-T cells of Example 2C in Table 13 would possess excellent cancer cell killing capabilities, with higher than 50% of T cells being CD8$^+$.

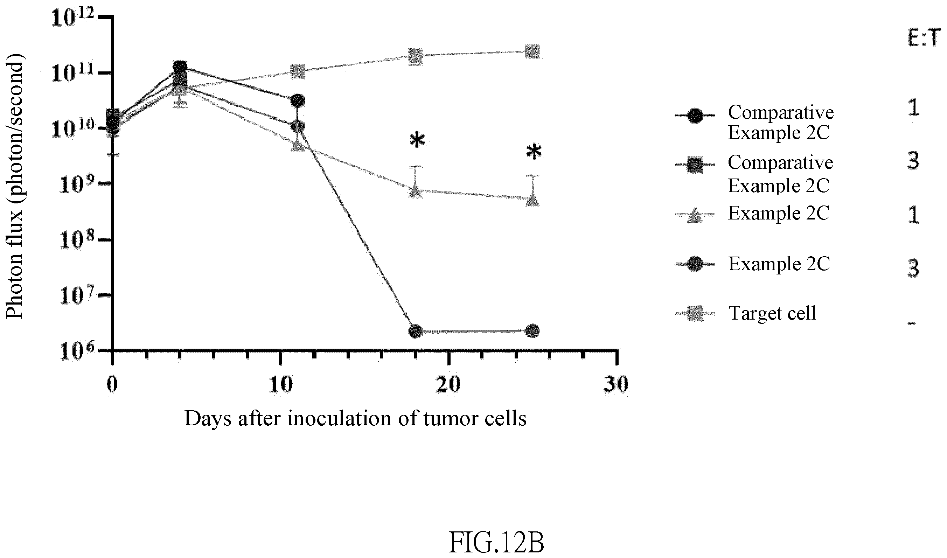

Substantial anti-tumor effects were observed in mice treated with low doses of CAR-T cells from Example 2C, following re-challenge of tumor cells (as shown in FIGs.12A and 12B). Moreover, except for the mice treated with low doses of CAR-T cells from Example 2C, most other mice treated with CAR-T cells experienced GvHD. In mice treated with CAR-T cells from Example 2C, the BLI signal intensities were reduced significantly to becoming no longer detectable visually, which represented significant anti-tumor effect of Example 2C, and on day 18 after tumor re-challenge, quantitative values of BLI were statistically lower than that of the control group with saline treatment.

Based on the results above, the lentivirus of Example 2B of the present invention, having an intracellular signaling domain and containing the NKp30 transmembrane domain (the fragment of NKp30 without immunoglobulin-like domain), demonstrates high transduction efficiency, and the resulting CAR-T cells exhibited a superior cell expansion rate, excellent cytotoxicity, and high proportions of Tscm cells, suggesting the potential to reduce cytokine release syndrome and neurotoxicity caused by CAR-T cell therapy. The animal experiment also verifies that the produced CAR-T cells have excellent anti-tumor efficacy, with less severe GvHD symptoms, and could potentially avoid fatal cytokine release syndrome (CRS) caused by over activation of traditional single-chain CAR-T cells. The hyperactive CAR-T cells exhaust easily leads to weaker persistency in body, resulting in unsustainable efficacy or even increasing likelihoods of recurrence according to the contents of literature: Korell F. et al, Med. 2022 Aug 12;3(8):538-564. It can be inferred that during the preparation of CAR-T cells, transducing a nucleic acid sequence encoding an intracellular signaling domain, such as the DAP12 intracellular signaling domain into T cells can significantly enhance the cytotoxicity of produced CAR-T cells.

### III. Preparation and test of CAR-T cells of anti-CD22-NKp30 which has an intracellular signaling domain

**Example 3: Nucleic acid molecule having intracellular signaling domain, scFv of anti-CD22 antibody and NKp30 fragment without immunoglobulin-like domain**

The 3' end of a nucleic acid sequence of full length FcεR1 $^{1\gamma\ (s]}$ (shown as SEQ ID NO: 77) was linked to the 5' end of a nucleic acid sequence of the DAP12 intracellular signaling domain (shown as SEQ ID NO: 78), to obtain a linked nucleic acid molecule fragment 1. Then, the 3' end of the linked nucleic acid molecule fragment 1 was linked to the 5' end of a nucleic acid sequence of m971 scFv of anti-CD22 antibody (shown as SEQ ID NO: 91) by a nucleic acid sequence of T2A self-cleaving peptide (shown as SEQ ID NO: 80). Then, the 5' end of a nucleic acid sequence of hinge domain of CD28 (shown as SEQ ID NO: 68) was linked to the 3' end of the nucleic acid sequence of m971 scFv of anti-CD22 antibody. Last, the 5' end of a nucleic acid sequence of NKp30 fragment without immunoglobulin-like domain (shown as SEQ ID NO: 86) was linked to the 3' end of the nucleic acid sequence of hinge domain of CD28, to obtain a nucleic acid molecule having intracellular signaling domain, scFv of anti-CD22 antibody and NKp30 of Example 3, which was shown as SEQ ID NO: 92. The nucleic acid molecule of Example 3 was, from the 5' end to the 3' end, the nucleic acid sequence of full length FcεR1

γ', , the nucleic acid sequence of DAP12 intracellular signaling domain, the nucleic acid sequence of T2A self-cleaving peptide, the nucleic acid sequence of m971, the nucleic acid sequence of the hinge domain of CD28 and the nucleic acid sequence of NKp30 fragment without immunoglobulin-like domain.

**Comparative Example 3-1: Nucleic acid molecule having intracellular signaling domain and scFv of anti-CD22 antibody, without any NKp30 fragment**

The preparation method of Comparative Example 3-1 was similar to the preparation method of Comparative Example 2, except that the single-chain variable fragment used in Comparative Example 3-1 was not SS1 scFv of anti-mesothelin of Comparative Example 2, but a nucleic acid sequence of m971 scFv of anti-CD22 antibody (shown as SEQ ID NO: 91) A nucleic acid sequence of the nucleic acid molecule of Comparative Example 3-1 is shown as SEQ ID NO: 93.

**Comparative Example 3-2: Nucleic acid molecule having scFv of anti-CD22 antibody and NKp30 fragment without immunoglobulin-like domain, and without intracellular signaling domain**

The 3' end of a nucleic acid sequence of full length FcεR1 γ' (shown as SEQ ID NO: 77) was linked to the 5' end of a nucleic acid sequence of m971 scFv of anti-CD22 antibody (shown as SEQ ID NO: 91) by a nucleic acid sequence of T2A self-cleaving peptide (shown as SEQ ID NO: 80). Then, the 5' end of a nucleic acid sequence of hinge domain of CD28 (shown as SEQ ID NO: 68) was linked to the 3' end of the nucleic acid sequence of m971 scFv of anti-CD22 antibody. Last, the 5' end of a nucleic acid of encoding NKp30 fragment without immunoglobulin-like domain (shown as SEQ ID NO: 86) was linked to the 3' end of the nucleic acid sequence of hinge domain of CD28, to obtain a nucleic acid molecule of anti-CD22-NKp30 without intracellular signaling domain of Comparative Example 3-2, which was shown as SEQ ID NO: 94.

The nucleic acid molecule of Comparative Example 3-2 was, from the 5' end to the 3' γ' end, the nucleic acid sequence of full length FcεR1, the nucleic acid sequence of T2A self-cleaving peptide, the nucleic acid sequence of m971, the nucleic acid sequence of the hinge domain of CD28 and the nucleic acid sequence of NKp30 fragment without immunoglobulin-like domain.

**Example 3A, Comparative Example 3-1A and Comparative Example 3-2A: Construction of plasmids with the nucleic acid molecules of Example 3, Comparative Example 3-1 and Comparative Example 3-2**

The preparation method of Example 3A was similar to the preparation methods of Example 1-1A and Example 2-1A, and the difference was that the nucleic acid molecule of Example 3 was ligated into the foresaid pLAS5w vector by restriction enzymes of HpaI and EcoRI in Example 3A.

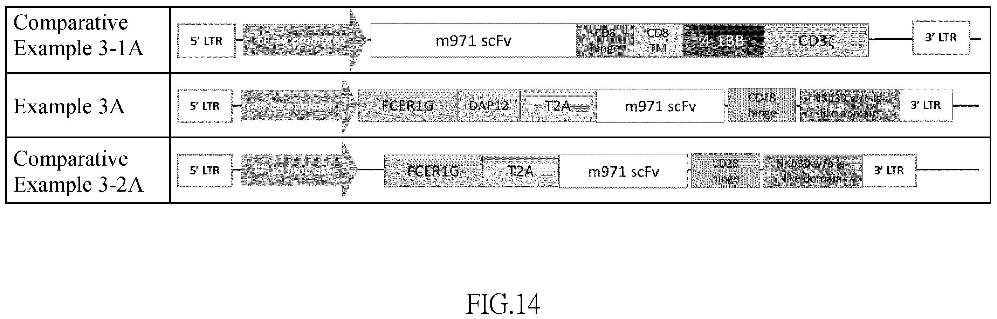

The nucleic acid molecule of Comparative Example 3-1 was ligated into pLAS5w.Ppuro vector (purchased from Academia Sinica RNAi Core facility, Product No.: C6-8-39) by restriction enzymes of NheI and EcoRI in Comparative Example 3-1A (conducted by GenScript). The nucleic acid molecule of Comparative Example 3-2 was ligated into the foresaid pLAS5w vector by restriction enzymes of HpaI and EcoRI in Comparative Example 3-2A. The prepared plasmids of Example 3A, Comparative Example 3-1A and Comparative Example 3-2A are shown in FIG.14.

**Example 3B, Comparative Example 3-1B and Comparative Example 3-2B: Preparation of lentiviruses with the nucleic acid molecules of Example 3, Comparative Example 3-1 and Comparative Example 3-2**

The preparation method of Example 3B was similar to the preparation methods of Examples 1-1B and 1-2B, and the difference was that the plasmid of Example 3A was adopted as the transfer plasmid for preparation of Example 3B. While, the plasmids of Comparative Example 3-1A and Comparative Example 3-2A were adopted as transfer plasmids for the lentivirus preparation of Comparative Example 3-1B and Comparative Example 3-2B, respectively.

**Test Example 13: Titer determination of lentivirus of Example 3B**

The method in Test Example 13 was similar to Test Example 1, and the difference was that Biotinylated Human CD22, Fc tag, (Sino Biological, Cat: 11958-H41H-B) was used to target anti-CD22 receptor and 500-fold diluted Streptavidin conjugated PE (Invitrogen) was used to detect the expressions of anti-CD22 receptor of lentiviruses of Example 3B and Comparative Example 3-1B. The test results are shown in the following Table 14.

Table 14: the functional viral titers of lentiviruses of Example 3B and Comparative Example 3-1B

| Group | Example 3B | Comparative Example 3-1B |
|---|---|---|
| Functional viral titer (TU/mL) | $1.3\times10^7$ | $1.2\times10^7$ |

When the functional viral titer is more than or equal to $1\times10^6$ TU/mL, when preparing CRT-T cells, T cells can be successfully transduced and become CAR-T cells with a lower amount of lentivirus, and the transduced T cells tended to have better growth. Therefore, according to the results in above Table 14, Example 3B met the foresaid criteria, which further benefited the subsequent preparation of CAR-T cells.

**Example 3C: Preparing CAR-T cells with the nucleic acid molecules of Example 3, Comparative Example 3-1 and Comparative Example 3-2**

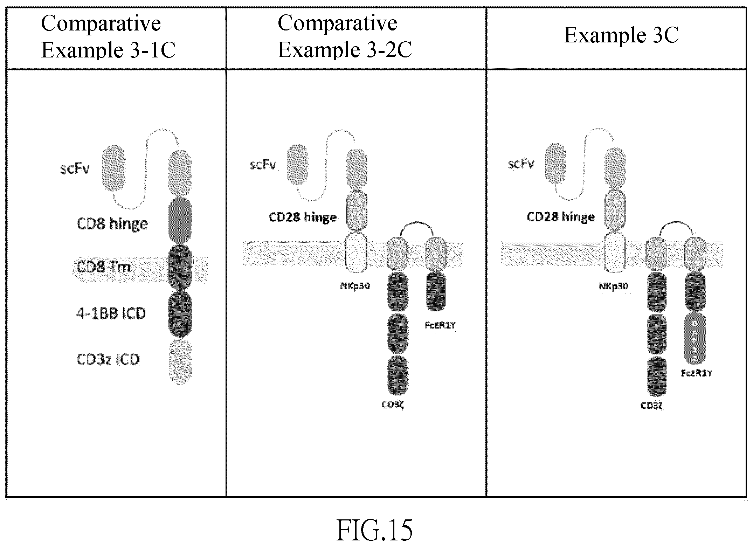

The preparation method of CAR-T cells of Example 3C was similar to the preparation methods of Examples 1-1C and 1-2C, and the difference was that the T cells were transduced with the lentivirus of Example 3B and the MOI of lentivirus transduction was 3 in this Test Example. While, CAR-T cells of Comparative Example 3-1C and Comparative Example 3-2C were prepared by transducing with the lentiviruses of Comparative Example 3-1B and Comparative Example 3-2B, and the MOI of lentivirus transduction was 3. The structural schematic illustrations of the prepared CAR-T cells of Example 3C and CAR-T cells of Comparative Example 2C are shown in FIG.15.

**Test Example 14: Cell expansion experiments of CAR-T cells of Example 3C, Comparative Example 3-1C and Comparative Example 3-2C, and T cells of the un-transduced group**

The method in Test Example 14 was similar to Test Example 2, and the difference was that the cell expansion rates of CAR-T cells of Example 3C, Comparative Example 3-1C and Comparative Example 3-2C were detected in this Test Example, and the un-transduced, activated T cells were set as a control group. The results are shown in the following Table 15.

Table 15: expansion folds on the 7th day of CAR-T cells of Example 3C, Comparative Example 3-1C and Comparative Example 3-2C, and T cells of the un-transduced group

| Group | Example 3C | Comparative Example 3-1C | Comparative Example 3-2C | Un-transduced group (control group) |
|---|---|---|---|---|
| Expansion folds on day 7 relative to day 0 | 28X | 30.15X | 24.5X | 39.67X |

When the expansion fold can reach more than 5-fold, it was beneficial to the subsequent functional tests of T cells. Therefore, according to the results in above Table 15, CAR-T cells of Example 3C performed superior T-cell expansion fold, and could be used in the subsequent functional test for T cells.

**Test Example 15: Expression results of CAR-T cells of Example 3C and Comparative Example 3-1C; transduction efficiencies of lentiviruses of Example 3B and Comparative Example 3-1B**

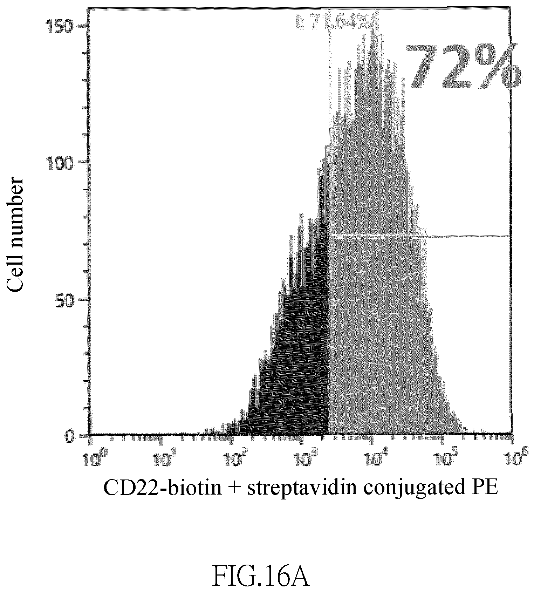

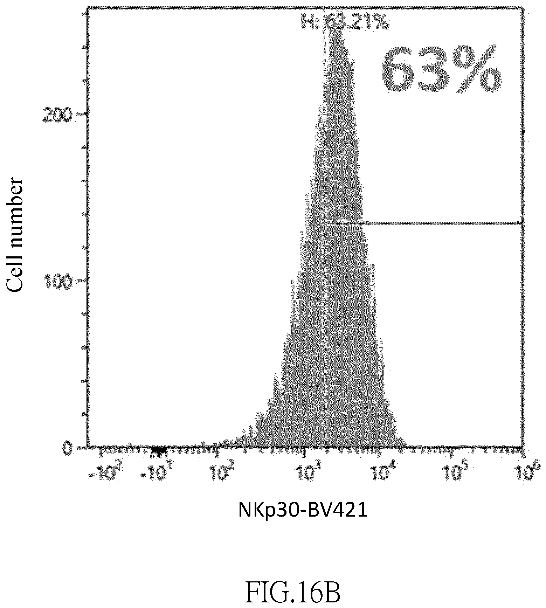

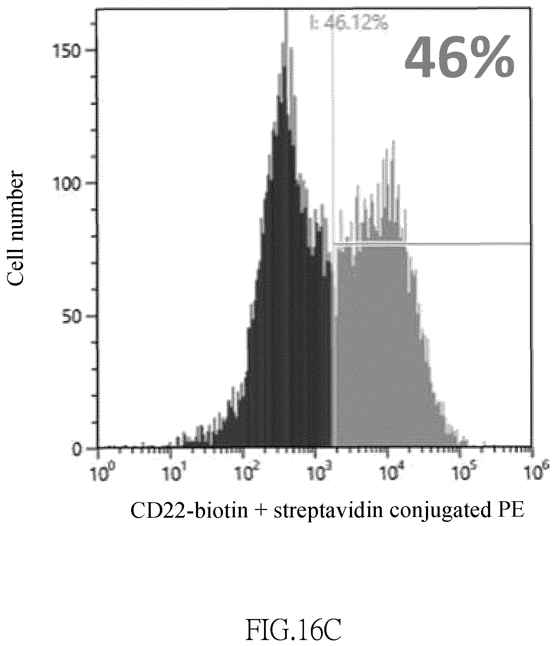

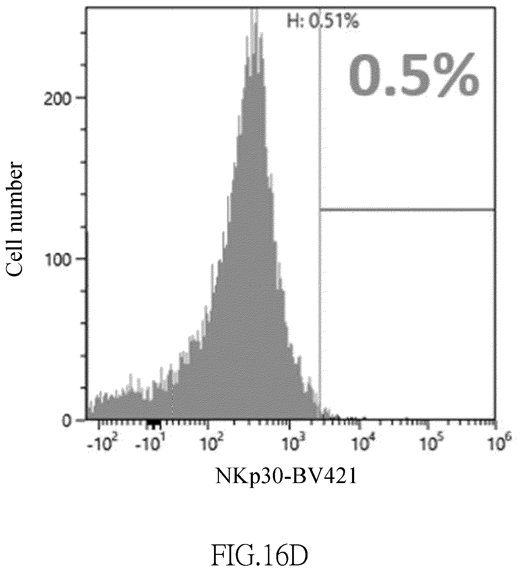

The method in Test Example 15 was similar to Test Example 3, and the difference was that Biotinylated Human CD22 protein, Fc Tag, (Sino Biological, Cat: 11958-H41H-B) was used to target anti-CD22 receptor and 500-fold diluted Streptavidin conjugated PE (Invitrogen) were used for detecting the expressions of anti-CD22 receptor of CAR-T cells of Example 3C and Comparative Example 3-1C. The results analyzed by a flow cytometer of Example 3C and Comparative Example 3-1C were shown in FIGs.16A, 16B, 16C and 16D, respectively. Besides, the transduction efficiencies of lentiviruses of Example 3B or Comparative Example 3-1B were the percentage of the number of cells expressing anti-CD22 receptor relative to the total cell number, and the experimental results were shown in the following Table 16.

Table 16: the transduction efficiencies of lentiviruses of Example 3B and Comparative Example 3-1B

| Group | Example 3B | Comparative Example 3-1B |
|---|---|---|
| Transduction efficiency | 40% to 80% | 30% to 75% |

When the transduction efficiency of preparing CAR-T cells was greater than 25%, it was beneficial to the subsequent functional tests of expanded CAR-T cells. Therefore, according to the results in above Table 16 and FIG.16A, the lentivirus of Example 3B exhibited high transduction efficiency.

**Test Example 16: T cell subpopulation analysis for CAR-T cells of Example 3C**

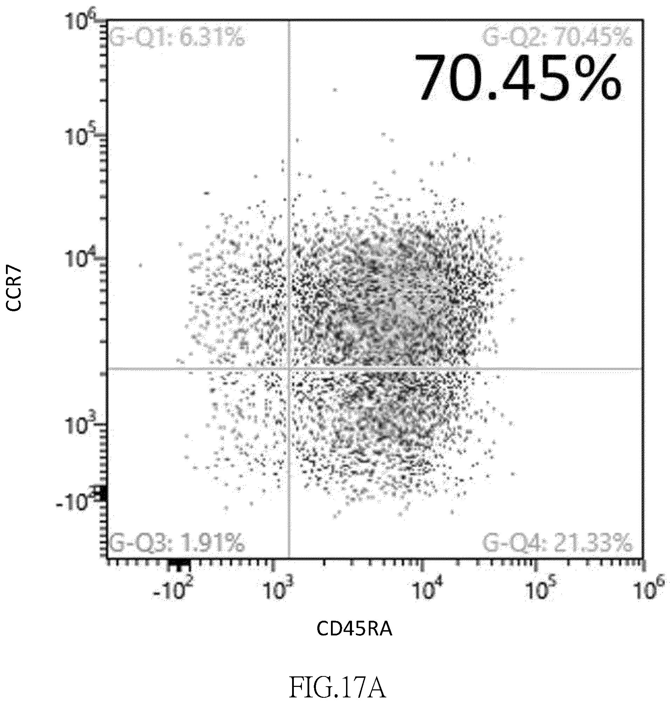

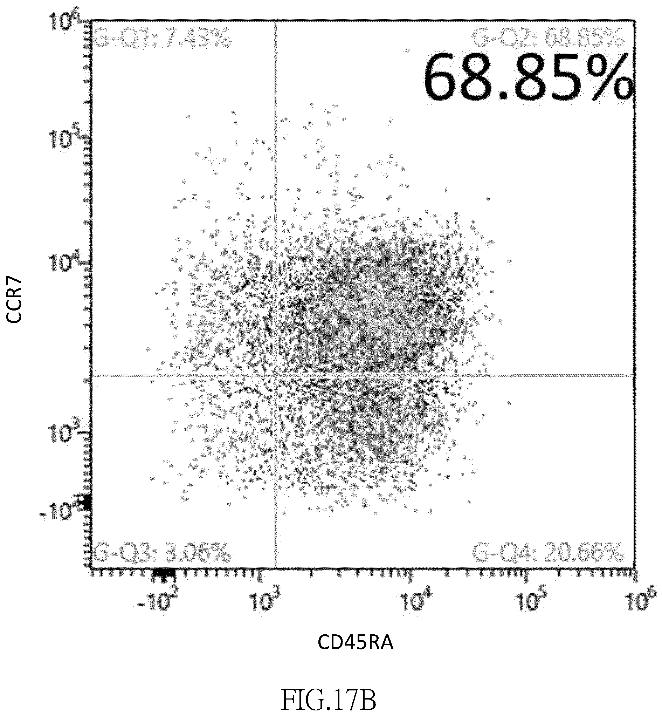

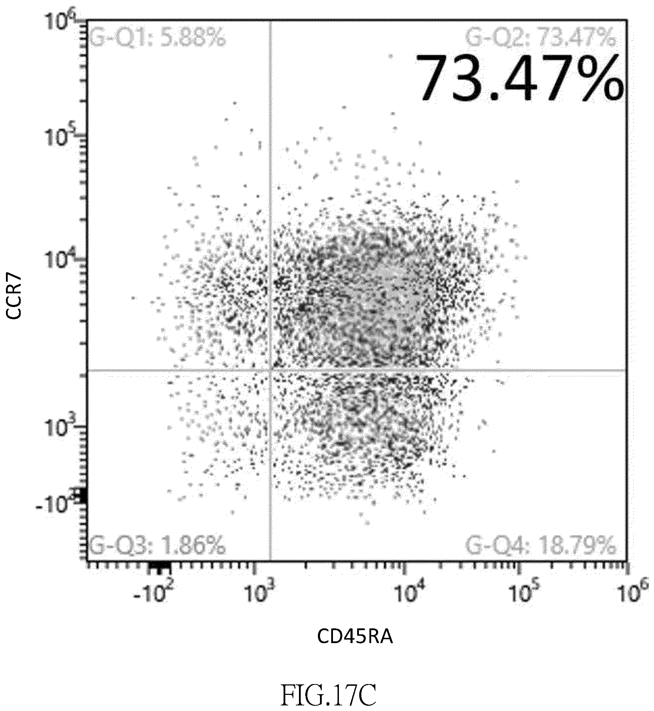

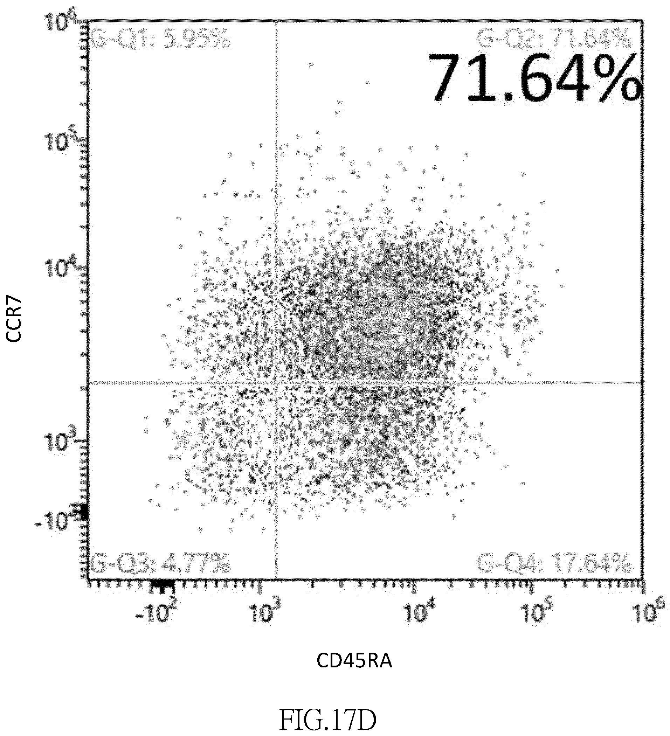

The method in Test Example 16 was similar to Test Example 4, and the difference was that the analyses were performed on the 7th day of the preparation of CAR-T cells of Example 3C, Comparative Example 3-1C and Comparative Example 3-2C in this Test Example. The percentages of Tscm in CAR-T cells of Example 3C, Comparative Example 3-1C and Comparative Example 3-2C, and in T cells of the un-transduced group (control group) were shown in the following Table 17; and the distribution of the subsets of T cells of Example 3C, Comparative Example 3-1C and Comparative Example 3-2C, and the un-transduced group from flow cytometry were shown in FIGs.17A, 17B, 17C and 17D, respectively; wherein, T cells expressing CD45RA and CCR7 were Tscm.

Table 17: the percentages of Tscm in CAR-T cells of Example 3C, Comparative Example 3-1C and Comparative Example3-2C, and the percentage of Tscm in T cells of the un-transduced group

| Group | Example 3C | Comparative Example 3-1C | Comparative Example 3-2C | Un-transduced group (control group) |
|---|---|---|---|---|
| Tscm(%) | 70.5% | 68.9% | 74.5% | 71.6% |

If the stem cell-like memory T cell percentage in CAR-T cells was greater than 40%, cytokine release syndrome and neurotoxicity caused by CAR-T cell therapy will be reduced. According to above Table 17 and FIGs.17A, 17B, 17C and 17D, Example 3C displayed higher proportions of Tscm, and was slightly better than traditional single-chain CAR-T cells of Comparative Example 3-1C, while similar to the activated T cells of the un-transduced group. Therefore, it could be expected that CAR-T cells of Example 3C were able to reduce cytokine release syndrome and neurotoxicity caused by CAR-T cell therapy.

**Test Example 17: Real-time cytotoxicity assay by using xCELLigence**

Human CD22 cDNA fragment was cloned from Raji human lymphoma cell line (purchased from Elabscience, Cat. No. CL-0189). The cDNA fragment of a truncated CD22 (shown as SEQ ID NO: 95) was ligated into a pLAS5w.Ppuro vector (purchased from Academia Sinica RNAi Core facility, Product No.: C6-8-39), to obtain a plasmid with the truncated CD22. The plasmid with the truncated CD22 was then transfected into SKOV3 human ovarian cancer cell line by using Polyjet transfection reagent (SignaGen Laboratories). The transfected SKOV3 cells were cultured in McCoy 5A Medium (Gibco) containing 2 μg/mL of puromycin and 10% FBS to select a SKOV3 cell line that was puromycin-resistant and expressed CD22 at the same time. At last, the expression of CD22 was re-checked by flow cytometry. The SKOV3 cell line expressing CD22 was used as target cells for the subsequent cytotoxicity assay conducted by using the xCELLigence system.

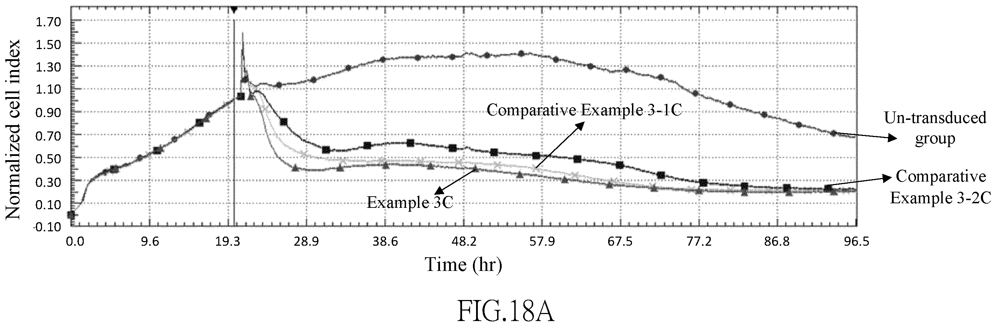

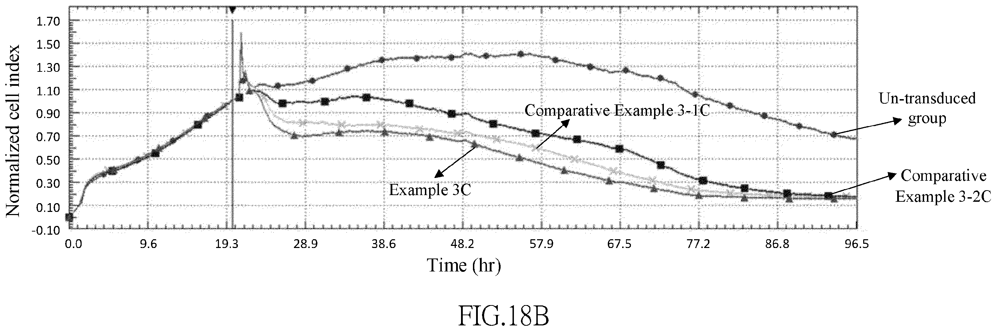

The experimental method of Test Example 17 was similar to Test Example 5, and the difference was that the effector cells were CAR-T cells of Example 3C, Comparative Example 3-1C and Comparative Example 3-2C in this Test Example, and the target cells were the foresaid SKOV3 cell line expressing CD22. To make the target cells attach in a culture plate with the background corrected and then the impedance measurement was performed for 20 hours. After overnight for complete cell adherence of the target cells, the effector cells were added into the culture plate at E:T ratios of 3:1 and 1:1 for testing. The results of cytotoxicity assay were respectively shown in FIGs.18A and 18B.

According to the results in FIGs.18A and 18B, the cytotoxicity ability of CAR-T of Example 3C was better than Comparative Example 3-1C which was the traditional single-chain CAR-T cells and Comparative Example 3-2C which had no intracellular signaling domain. When the cytotoxicity of CAR-T cells exceeds 50%, CAR-T produced using patient's cell should also exhibit sufficient cytotoxicity. According to the results in FIGs.18A and 18B, CAR-T cells of Example 3C demonstrated excellent cytotoxicity effect, which was better than CAR-T cells of Comparative Examples 3-1C and 3-2C.

**Test Example 18: cytotoxicity assay to human lymphoma cells analyzed by flow cytometer**

In order to test the anti-tumor activity of CAR-T cells of anti-CD22-NKp30 which containing an intracellular signaling domain of Example 3C. Human lymphoma cells (hereinafter referred to as Daudi cells) were labeled by CellTrace™ Violet in this Test Example, and then were cultured in RPMI-1640 medium (Gibco) containing 10% FBS for the subsequent cytotoxicity assay analyzed by flow cytometer. CAR-T cells of Example 3C, Comparative Example 3-1C and Comparative Example 3-2C were used as effector cells in this Test Example, and the un-transduced T cells were used as a control group. Specifically, the target cells with a cell density of $1.2 \times 10^4$ cells/well were seeded in a 96-well plate, followed by the addition of the effector cells to make the effector cell to target cell ratio (E:T) be 1:1 and 3:1, and then cultured at 37°C in 5% $CO_2$ for 24 hours, and with the experiment performed in triplicates. Next, the 96-well plates were centrifuged at $300 \times g$ for 5 minutes and then the supernatant was removed. Cells in each well were washed with 200 μL of PBS containing 2% fetal calf serum (FCS). After removing PBS for wash, 300 μL of flow buffer (PBS containing 2% FCS) was applied to collect cells. After 15 μL of counting microbeads (CountBright™ Plus Absolute Counting Beads, Invitrogen, C36995) were added, the number of the target cells was calculated by collecting 3000 microbeads with a flow cytometer for calculation. Cytotoxicity percentage was calculated according to the following equation:

cytotoxicity percentage (%) = (number of the target cells in the group without adding the effector cells - number of target cells after adding the effector cells for co-culture) / number of the target cells in the group without adding the effector cells $\times$ 100%.

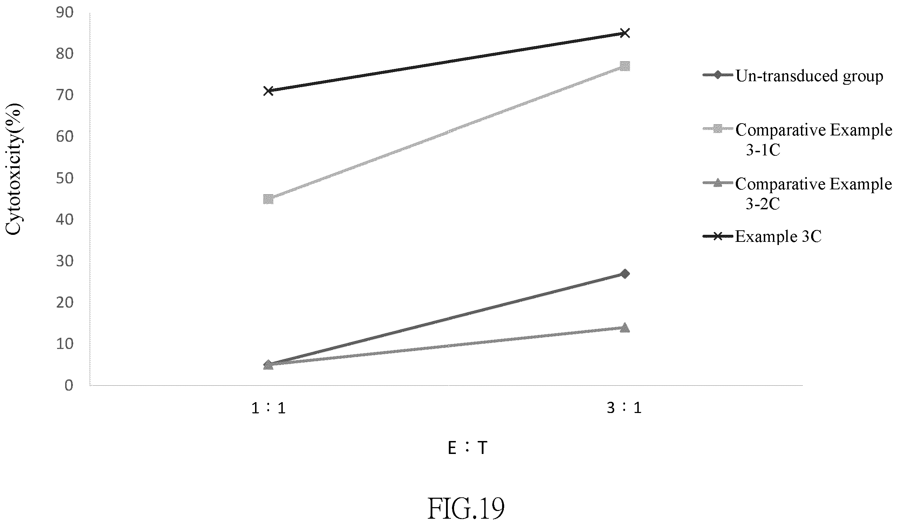

The experimental results were shown in FIG.19 and the following Table 18 with E:T ratio=3:1.

Table 18: The results of cytotoxicity assay to human lymphoma cells analyzed by flow cytometer of Example 3C, Comparative Example 3-1C and Comparative Example 3-2C

| Group | Example 3C | Comparative Example 3-1C | Comparative Example 3-2C |
|---|---|---|---|
| Specific lysis (E:T=3:1) | 85% | 77% | 14% |

According to the results in FIG.19 and Table 18, at E:T ratio of 1:1, the cytotoxicity effect of CAR-T cells of Example 3C was 71%, which was much better than 45% of Comparative Example 3-1C, which was single-chain CAR-T cells, 5% of Comparative Example 3-2C, which had no intracellular signaling domain, and 5% of the control group (un-transduce group). At E:T ratio of 3:1, the cytotoxicity effect of CAR-T cells of Example 3C was 85%, which was better than 77% of Comparative Example 3-1C which was traditional single-chain CAR-T cells, 14% of Comparative Example 3-2C which had no intracellular signaling domain, and 27% of the control group (un-transduce group). According to the comparison result between Example 3C and Comparative Example 3-2C, it could be observed that the cytotoxicity effect to cancer cells could be increased obviously when the intracellular signaling domain was added.

From above results, it is found that the lentivirus of Example 3B of the present invention has intracellular signaling domain and NKp30 transmembrane domain (NKp30 fragment without immunoglobulin-like domain), thereby demonstrating high transduction efficiency; and the produced CAR-T cells after transduction had superior cell expansion rate and excellent cytotoxicity, and higher proportions of Tscm, which could be expected to reduce cytokine release syndrome and neurotoxicity caused by CAR-T cell therapy. It can be inferred that when preparing CAR-T cells, if a nucleic acid sequence of intracellular signaling domain, such as DAP12 intracellular signaling domain, is transduced, it can bring excellent cytotoxicity effect for the produced CAR-T cells, and is especially suitable for CAR-T cell therapy.

**IV. Preparation and test of CAR-T cells of anti-CD19-NKp30 which contained an intracellular signaling domain**

**Example 4: Nucleic acid molecule having intracellular signaling domain, scFv of anti-CD19 antibody and NKp30 fragment without immunoglobulin-like domain**

The preparation method of Example 4 was similar to the preparation method of Example 3, and the difference was that the single-chain variable fragment used in Example 4 was not m971 scFv of anti-CD22 of Example 3, but a nucleic acid sequence of FMC63 scFv of anti-CD19 antibody (shown as SEQ ID NO: 96), and the adopted hinge domain was the hinge domain of human IgG4 (shown as SEQ ID NO: 69). A nucleic acid sequence of the nucleic acid molecule of Example 4 is shown as SEQ ID NO: 97. The nucleic acid molecule of Example 4 was, from the 5' end to the 3' end, the nucleic acid sequence of full length FcεR1$^{γ,}$, the nucleic acid sequence of the DAP12 intracellular signaling domain, the nucleic acid sequence of T2A self-cleaving peptide, the nucleic acid sequence of FMC63, the nucleic acid sequence of the hinge domain of IgG4 and the nucleic acid sequence of NKp30 fragment without immunoglobulin-like domain.

**Comparative Example 4: Nucleic acid molecule having intracellular signaling domain and scFv of anti-CD19 antibody, without any NKp30 fragment**

The preparation method of Comparative Example 4 was similar to the preparation method of Comparative Example 2, and the difference was that the single-chain variable fragment used in Comparative Example 4 was not SS1 scFv of anti-mesothelin of Comparative Example 2, but a nucleic acid sequence of FMC63 scFv of anti-CD19 antibody (shown as SEQ ID NO: 96). A nucleic acid sequence of the prepared nucleic acid molecule of Comparative Example 4 is shown as SEQ ID NO: 98.

**Example 4A: Construction of plasmids with the nucleic acid molecules of Example 4 or Comparative Example 4**

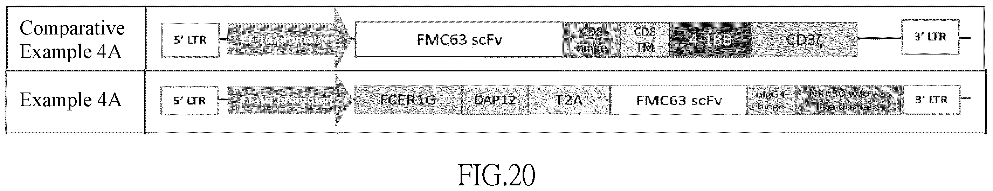

The preparation method of Example 4A was similar to the preparation methods of Examples 1-1A and 1-2A, and the difference was that the nucleic acid molecule of anti-CD19-NKp30 which has an intracellular signaling domain of Example 4 was ligated into the foresaid pLAS5w vector by restriction enzymes of HpaI and EcoR1 in Example 4A.
The nucleic acid molecule of Comparative Example 4 was ligated into pLAS5w.Ppuro vector (purchased from Academia Sinica RNAi Core facility, Product No.: C6-8-39) by restriction enzymes of NheI and EcoR in Comparative Example 4 (conducted by GenScript). The plasmids of Example 4A and Comparative Example 4A prepared according to the foresaid preparation methods were shown in FIG.20.

**Example 4B and Comparative Example 4B: Preparation of lentiviruses with the nucleic acid molecules of Example 4 or Comparative Example 4**

The preparation method of Example 4B was similar to the preparation methods of Examples 1-1B and 1-2B, and the difference was that the plasmid of Example 4A was adopted as the transfer plasmid for preparation of Example 4B. While, the plasmid of Comparative Example 4A was adopted as transfer plasmids for the lentivirus preparation of Comparative Example 4B.

**Test Example 19: Titer determination of lentiviruses of Example 4B and Comparative Example 4B**

The method in Test Example 19 was similar to Test Example 1, and the difference was that Biotinylated Human CD19, Fc tag (ACROBiosystems, CD9-H8259) was used to target anti-CD19 receptor and 500-fold diluted Streptavidin conjugated PE (Invitrogen) was used to detect the expressions of anti-CD19 receptor of lentiviruses of Example 4B and Comparative Example 4B. The test results are shown in the following Table 19.

Table 19: the functional viral titers (transducing unit, TU / mL) of lentiviruses of Example 4B and Comparative Example 4B

| Group | Example 4B | Comparative Example 4B |
|---|---|---|
| Functional viral titer (TU/mL) | $1\times10^7$ to $1\times10^8$ | $2\times10^6$ to $3\times10^6$ |

When the functional viral titer was more than or equal to $1\times10^6$ TU/mL, when preparing CAR-T cells, T cells can be successfully transduced and become CAR-T cells with a lower amount of lentivirus, and the transduced T cells tended to have better growth. Therefore, according to the results in above Table 19, the lentivirus of Example 4B met the foresaid criteria, which further benefited the subsequent preparation of CAR-T cells.

**Example 4C and Comparative Example 4C: Preparing CAR-T cells with the nucleic acid molecules of Example 4 and Comparative Example 4**

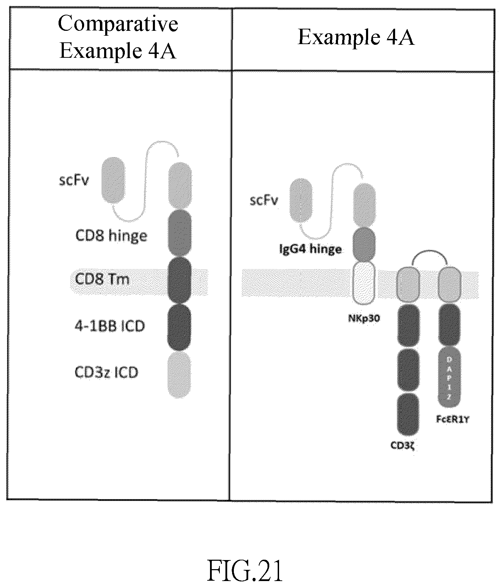

The preparation method of CAR-T cells of Example 4C was similar to the preparation methods of Examples 1-1C and 1-2C, and the difference was that the T cells were transduced with the lentivirus of Example 4B and the MOI of lentivirus transduction was 2 in this Test Example. While, CAR-T cells of Comparative Example 4C were transduced with the lentivirus of Comparative Example 4C, and the MOI of lentivirus transduction was 2. The structural schematic illustrations of the prepared CAR-T cells of Example 4C and CAR-T cells of Comparative Example 4C are shown in FIG.21.

**Test Example 20: Cell expansion experiments of CAR-T cells of Example 4C and Comparative Example 4C**

The method in this Test Example was similar to Test Example 2, and the difference was that the cell expansion rates of CAR-T cells of Example 4C and Comparative Example 4C were detected in this Test Example, and the un-transduced, activated T cells were set as a control group (un-transduced group). The results are shown in the following Table 20.

Table 20: expansion folds on the 7th day of CAR-T cells of Example 4C and Comparative Example 4C

| Group | Example 4C | Comparative Example 4C | Un-transduced group (control group) |
|---|---|---|---|
| Expansion folds on day 7 relative to day 0 | 14.17X | 10.34X | 10X |

When the expansion fold can reach greater than 5-fold, it was beneficial to the subsequent functional tests of T cells. Therefore, according to the results in above Table 20, CAR-T cells of Example 4C had superior expansion fold for T cells, and could be used in the subsequent functional test for T cells.

**Test Example 21: Expression detection of CAR-T cells of Example 4C and Comparative Example 4C; transduction efficiencies of lentiviruses of Example 4B and Comparative Example 4C**

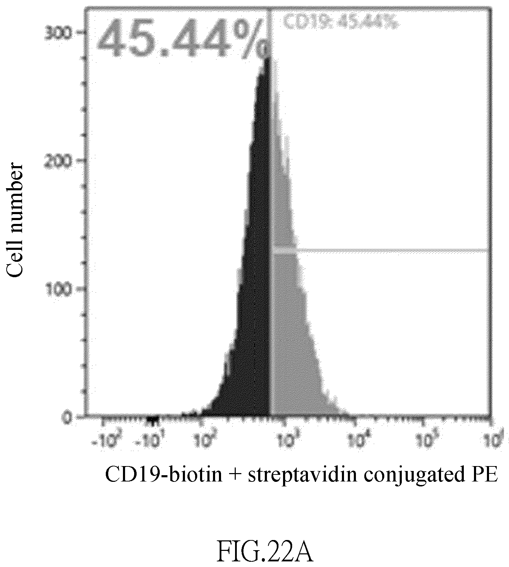

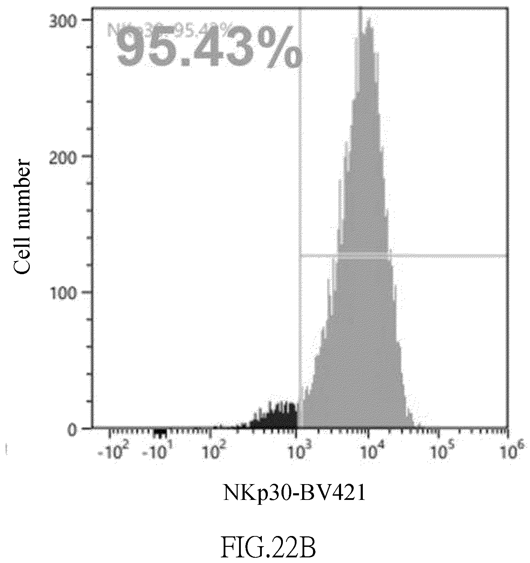

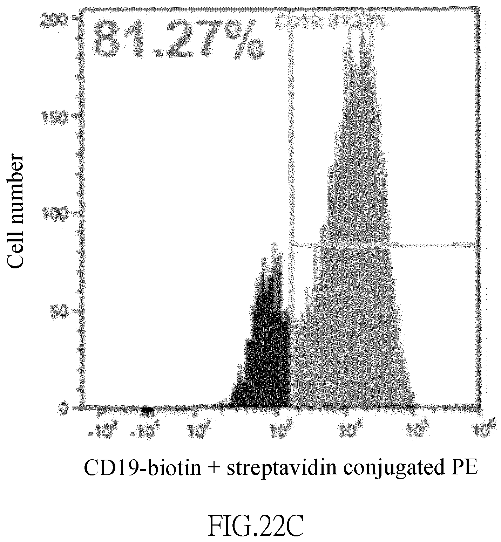

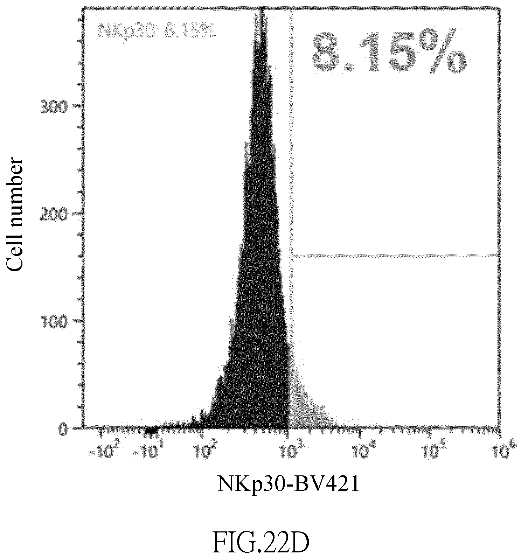

The method in Test Example 21 was similar to Test Example 3, and the difference was that Biotinylated Human CD19, Fc Tag (ACROBiosystems, CD9-H8259) was used to target anti-CD19 receptor and 500-fold diluted Streptavidin conjugated PE (Invitrogen) was used to detect the expressions of anti-CD19 receptor of CAR-T cells of Example 4C and Comparative Example 4C. The test results of Example 4C and Comparative Example 4C were respectively shown as the flow cytometry results in FIGs.22A, 22B, 22C and 22D. Then, the transduction efficiencies of lentiviruses of Example 4B or Comparative Example 4B were the percentage of the number of cells expressing anti-CD19 receptor relative to the total cell number, and the experimental results were shown in the following Table 21.

Table 21: the transduction efficiencies of lentiviruses of Example 4B and Comparative Example 4B

| Group | Example 4B | Comparative Example 4B |
|---|---|---|
| Transduction efficiency | 30% to 50% | 30% to 85% |

When the transduction efficiency of preparing CAR-T cells is greater than 25%, it is beneficial to the subsequent functional tests of expanded CAR-T cells. Therefore, according to the results in above Table 21 and FIG.22A, the lentivirus of Example 4B exhibited high transduction efficiency.

**Test Example 22: T cell subpopulation analysis for CAR-T cells of Example 4C**

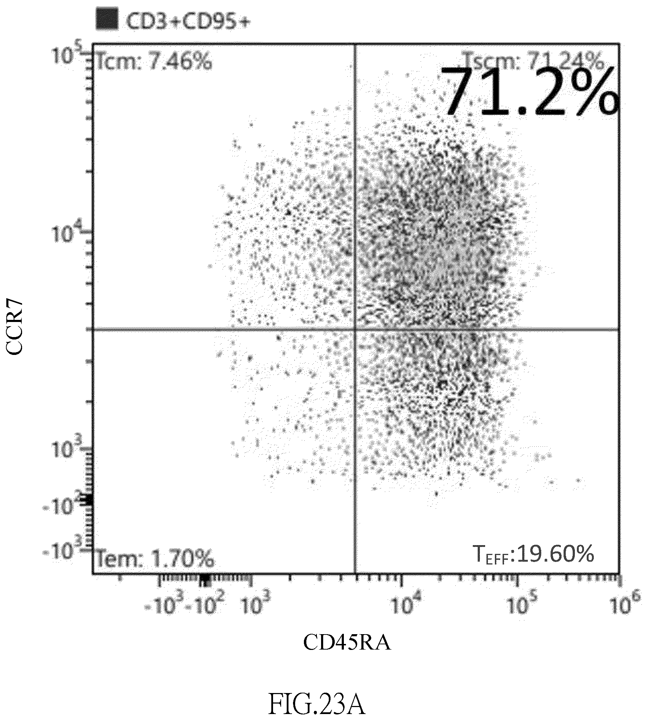

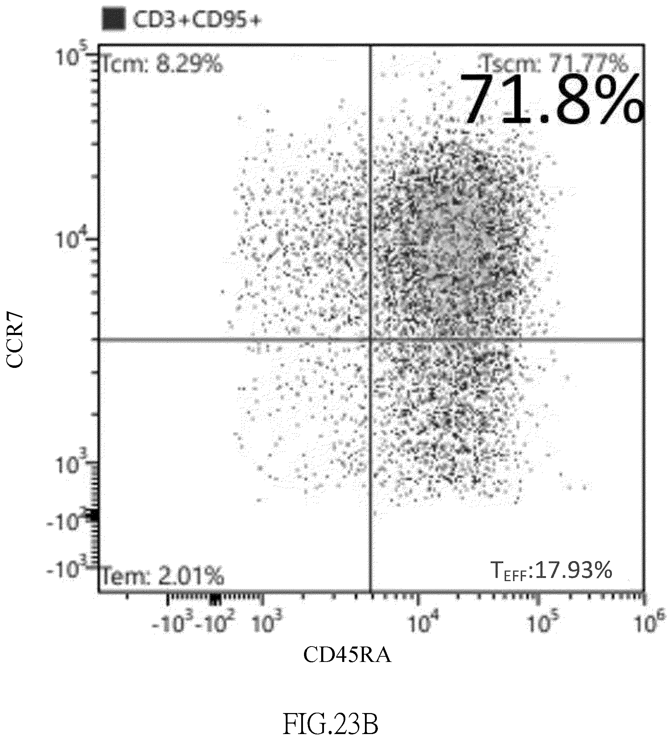

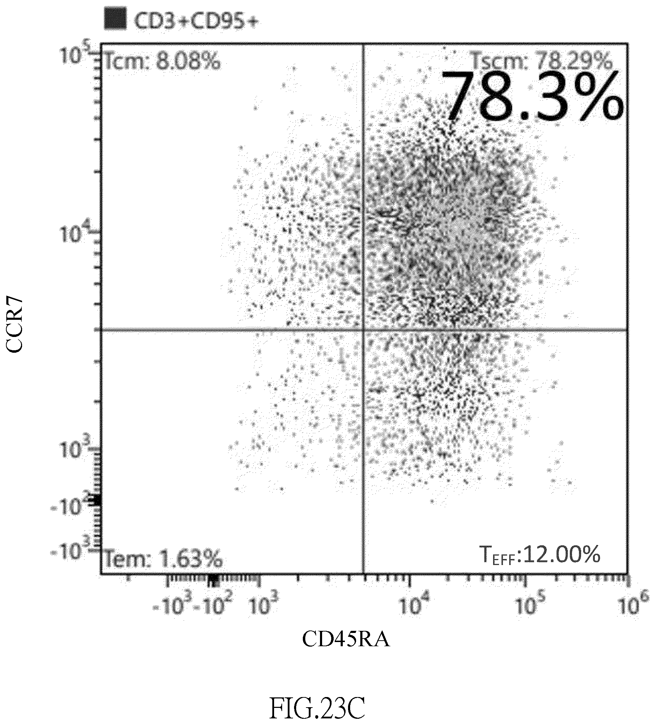

The method in Test Example 22 was similar to Test Example 4, and the difference was that the analyses were performed on the 7th day of the preparation of CAR-T cells of Example 4C in this Test Example. The percentages of Tscm in CAR-T cells of Example 4C and Comparative Example 4C, and in T cells of the un-transduced group are shown in the following Table 22; and the results of the distribution of the subpopulations of T cells of Example 4C result and Comparative Example 4C, and the un-transduced group from flow cytometry are shown in FIGs.23A, 23B and 23C, respectively; wherein, T cells simultaneously expressing CD45RA and CCR7 were considered Tscm.

Table 22: the percentages of Tscm in CAR-T cells of Example 4C and Comparative Example 4C, and the percentage of Tscm in T cells of the un-transduced group

| Group | Example 4C | Comparative Example 4C | Un-transduced group |
|---|---|---|---|
| Tscm(%) | 71.2% | 71.8% | 78.3% |

When the percentage of Tscm in CAR-T cells is greater than 40%, cytokine release syndrome and neurotoxicity caused by CAR-T cell therapy can be reduced. According to above Table 22 and FIGs.23A, 23B and 23C, Example 4C displayed proportions of Tscm higher than 40%, and was similar to traditional single-chain CAR-T cells, which could be expected to reduce cytokine release syndrome and neurotoxicity caused by CAR-T cell therapy.

**Test Example 23: Real-time cytotoxicity assay by using xCELLigence**

Human CD19 cDNA fragment was cloned from Raji human lymphoma cell line. The cDNA fragment of a truncated CD19 (shown as SEQ ID NO: 99) was amplified by PCR; wherein, the foresaid truncated CD19 cDNA fragment (amino acid 1 to 342) indicated the cDNA fragment having extracellular and transmembrane domains but lacking intracellular domain and cytoplasmic signaling domains. The foresaid truncated CD19 cDNA fragment was ligated into a pLAS5w.Ppuro vector, containing an anti-puromycin gene (purchased from Academia Sinica RNAi Core facility, Product No.: C6-8-39), using restriction enzyme sites of HpaI and NheI to obtain a plasmid with the truncated CD19. The plasmid with the truncated CD19 was then transfected into SKOV3 human ovarian cancer cell line by using Polyjet transfection reagent (SignaGen Laboratories, SL100688). The transfected SKOV3 cells were cultured in McCoy 5A Medium (Gibco) containing 2 $\mu$g/mL of puromycin and 10% FBS to select a SKOV3 cell line that was puromycin-resistant and expressed CD19 at the same time. At last, the expression of CD19 was re-checked by flow cytometry. The SKOV3 cell line expressing CD19 was used as target cells for the subsequent cytotoxicity assay conducted by using the xCELLigence system.

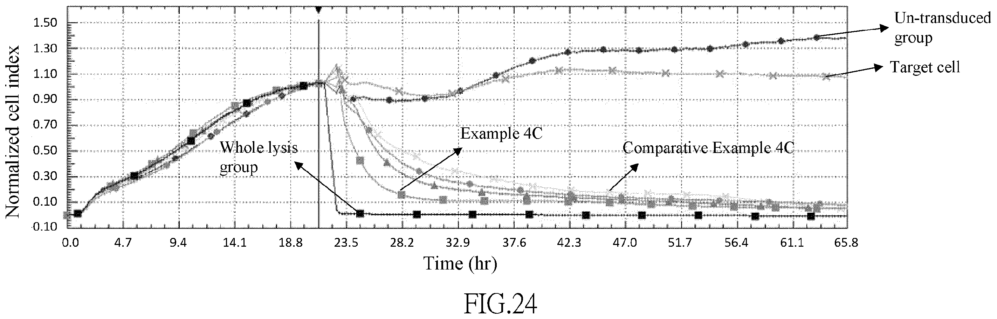

The experimental method in this Test Example was similar to Test Example 5, and the difference was that the effector cells were CAR-T cells of Example 4C and Comparative Example 4C in this Test Example, and the target cells were the foresaid SKOV3 cell line expressing CD19. The target cells were seeded in a culture plate with the background corrected, followed by 20 hours of overnight culture to make the target cells attach. Then, the effector cells were added into the microplate at an E:T ratio of 1:1 for testing. In addition, the culture medium cultured with $2\times10^4$ cells/mL of target cells and added with 100 $\mu$L of 1% Triton X-100 was used as a whole lysis group. The results of cytotoxicity assay were respectively shown in FIG.24, and the results of cytotoxicity assay for 48 hours were shown in Table 23.

Table 23: The results of cytotoxicity assay of Example 4C and Comparative Example 4C

| Group | Example 4C | Comparative Example 4C |
|---|---|---|
| Specific lysis | 92% | 84.4% |

When the cytotoxicity of CAR-T cells exceeds 50%, CAR-T produced using patient's cells should also exhibit sufficient cytotoxicity. According to the results in FIG.24 and Table 23, after CAR-T of Example 4C was co-cultured with the target cells for 48 hours, the cytotoxicity effect could reach higher than 80%, and was slightly better than the cytotoxicity effect of CAR-T cells of traditional single-chain CAR-T cells of Comparative Examples 4C. Therefore, it could be speculated that CAR-T cells of Example 4C had sufficient cytotoxicity when it is later changed to a patient's cells for CAR-T preparation.

**Test Example 24: Luciferase killing assay**

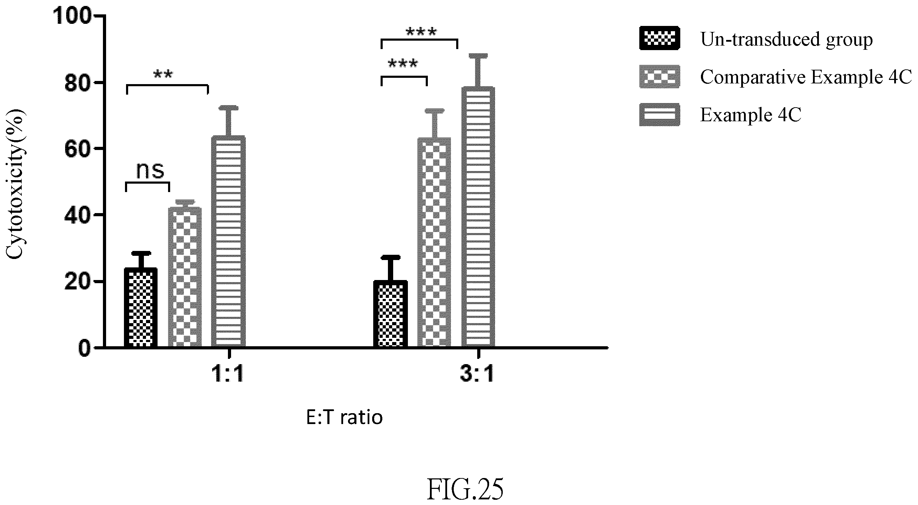

The experimental method in Test Example 24 was similar to Test Example 18, and the difference was that CAR-T cells of Example 4C and Comparative Example 4C were used as effector cells in this Test Example, and the foresaid Raji cell line expressing green fluorescent protein and luciferase enzyme was used as target cells to make the effector cell and target cells. Specifically, the effector cells and the target cells were co-cultured at 37°C in 5% $CO_2$ for 24 hours with an effector-to-target ratio (E:T ratio) of 1:1 and 3:1. The results of cytotoxicity were shown in FIG.25.

As the experimental results shown in FIG.25, regardless that E:T ratios was at 1:1 or 3:1, the cytotoxicity effect of CAR-T cells of Example 4C was higher than 50% and obviously better than the cytotoxicity effect of the single-chain CAR-T cells of Comparative Example 4C.

According to the foresaid results, the lentivirus of Example 4B of the present invention has intracellular signaling domain and NKp30 fragment with NKp30 transmembrane domain but without immunoglobulin-like domain, thereby demonstrating high transduction efficiency; and the produced CAR-T cells after transduction had superior cell expansion rate and excellent cytotoxicity, and higher proportions of Tscm, which could be expected to reduce cytokine release syndrome and neurotoxicity caused by CAR-T cell therapy. It can be concluded that introducing a nucleic acid sequence encoding for an intracellular signaling domain, such as DAP12, during CAR-T cell preparation can improve their cytotoxicity, offering significant benefits for CAR-T cell therapy.

In summary, transducing genes encoding intracellular signaling domains, such as DAP12, 41BB or CD28 intracellular signaling domain, along with the NKp30 transmembrane and cytoplasmic domain into T cells enables the formation of a multi-chain CAR-T cell with an NKp30 receptor complex, it can perform excellent killing ability to cancer cells through cytotoxicity in both *in vitro* and i*n vivo* studies.

The specific embodiments described above further describe the purpose, technical solutions and beneficial effects of the present invention in detail. It should be understood that the foresaid descriptions are only specific embodiments of the present invention and are not intended to limit the present invention. Within the spirit and principles of the present invention, any modifications, equivalent substitutions, improvements, etc. shall be included in the claimed scope of the present invention.

**Claims**

1. An isolated nucleic acid molecule comprising nucleic acid sequences of the following parts:

    (a) an extracellular antigen binding domain;
    (b) a hinge domain;
    (c) a NKp30 transmembrane domain;
    (d) a NKp30 cytoplasmic domain; and
    (e) an intracellular signaling domain.

2. The isolated nucleic acid molecule as claimed in claim 1, wherein the extracellular antigen binding domain comprises a heavy chain variable region.

3. The isolated nucleic acid molecule as claimed in claim 2, wherein the extracellular antigen binding domain comprises a heavy chain variable region of anti-mesothelin antibody, a heavy chain variable region of anti-CD22 antibody, a heavy chain variable region of anti-CD19 antibody, a heavy chain variable region of anti-BCMA antibody, a heavy chain variable region of anti-CD123 antibody, a heavy chain variable region of anti-GPRC5D antibody or a heavy chain variable region of anti-TSHR antibody.

4. The isolated nucleic acid molecule as claimed in claim 1, wherein the hinge domain is selected from the group consisting of: $(G_4S)_3$, a hinge domain of CD8, a hinge domain of CD28, a hinge domain of IgG4, EAAAKGGGGS, $(EAAAK)_3$, a hinge domain of GST, a hinge domain of EAAAK-GS, a hinge domain of IgD, and an IgG4-CH3 domain and $(AP)_6$.

5. The isolated nucleic acid molecule as claimed in claim 1, wherein the intracellular signaling domain is intracellular signaling domains of DAP12, 41BB or CD28.

6. The isolated nucleic acid molecule as claimed in claim 1, wherein the hinge domain and the NKp30 transmembrane domain are linked by a NKp30 extracellular stalk domain.

7. The isolated nucleic acid molecule as claimed in claim 1, wherein the extracellular antigen binding domain comprises a heavy chain variable region of anti-mesothelin antibody and the hinge domain is $(G_4S)_3$; the extracellular antigen binding domain comprises a heavy chain variable region of anti-CD22 antibody and the hinge domain is the hinge domain of CD28; the extracellular antigen binding domain comprises a heavy chain variable region of anti-CD19 antibody and the hinge domain is the hinge domain of IgG4.

8. The isolated nucleic acid molecule as claimed in claim 1, further comprising a nucleic acid sequence of an adapter.

9. A plasmid comprising the isolated nucleic acid molecule as claimed in any one of claims 1 to 8, wherein the plasmid is a DNA plasmid or an RNA plasmid.

10. A method for preparing a chimeric antigen receptor cell and the method comprising introducing the isolated nucleic acid molecule as claimed in any one of claims 1 to 8 into a nucleated cell.

11. The method as claimed in claim 10, wherein the isolated nucleic acid molecule is introduced into the nucleated cell by lentivirus transduction.

12. An isolated cell comprising the isolated nucleic acid molecule as claimed in any one of claims 1 to 8.

13. The isolated cell as claimed in claim 12, wherein the isolated cell is a T cell, a natural killer cell, a natural killer T cell or an adipose-derived stem cell.

14. A use of the isolated cell as claimed in claims 12 or 13 to prepare a medication for treating or alleviating cancer.

15. A use of the isolated cell as claimed in claims 12 or 13 to prepare a medication for treating or alleviating an autoimmune disease, wherein the autoimmune disease comprises systemic lupus erythematosus, idiopathic inflammatory myositis, systemic sclerosis or multiple sclerosis.

pLAS5w
7575 bp

FIG.1

| | | | | | | | | |
|---|---|---|---|---|---|---|---|---|
| Example 1-1A | 5' LTR | EF-1α promoter | FCER1G | DAP12 | T2A | NKp30 | 3' LTR | |
| Example 1-2A | 5' LTR | EF-1α promoter | FCER1G | 4-1BB | T2A | NKp30 | 3' LTR | |
| Comparative Example 1A | 5' LTR | EF-1α promoter | FCER1G | T2A | NKp30 | | 3' LTR | |

FIG.2

FIG.3

FIG.4A

FIG.4B

FIG.4C

FIG.4D

FIG.5A

FIG.5B

FIG.5C

| Comparative Example 2A | 5' LTR | EF-1α promoter | SS1scFv | CD8 hinge | CD8 TM | 4-1BB | CD3ζ | 3' LTR |
| Example 2A | 5' LTR | EF-1α promoter | FCER1G | T2A | SS1 scFv | (G₄S)₃ | NKp30 w/o like domain | DAP12 | 3' LTR |

FIG.6

EP 4 786 599 A1

FIG.7

FIG.8A

FIG.8B

SS1-biotin + streptavidin conjugated PE

FIG.8C

FIG.8D

FIG.9A

FIG.9B

44

FIG.9C

FIG.10A



FIG.10B

FIG.10C

FIG.10D

FIG.11A

EP 4 786 599 A1

FIG.11B

FIG.12A

E:T

Comparative Example 2C — 1

Comparative Example 2C — 3

Example 2C — 1

Example 2C — 3

Target cell — -

Photon flux (photon/second)

$10^{12}$ $10^{11}$ $10^{10}$ $10^{9}$ $10^{8}$ $10^{7}$ $10^{6}$

0 10 20 30

Days after inoculation of tumor cells

FIG.12B

53

FIG.13A

EP 4 786 599 A1

FIG.13B

| Comparative Example 3-1A | 5' LTR — EF-1α promoter → m971 scFv | CD8 hinge | CD8 TM | 4-1BB | CD3ζ | 3' LTR |
| Example 3A | 5' LTR — EF-1α promoter → FCER1G | DAP12 | T2A | m971 scFv | CD28 hinge | NKp30 w/o Ig-like domain | 3' LTR |
| Comparative Example 3-2A | 5' LTR — EF-1α promoter → FCER1G | T2A | m971 scFv | CD28 hinge | NKp30 w/o Ig-like domain | 3' LTR |

FIG.14

FIG.15

FIG.16A

FIG.16B

FIG.16C

FIG.16D

FIG.17A

FIG.17B

FIG.17C

FIG.17D

FIG.18A

FIG.18B

EP 4 786 599 A1

FIG.19

FIG.20

| Comparative Example 4A | Example 4A |
|---|---|

FIG.21

CD19-biotin + streptavidin conjugated PE

FIG.22A

FIG.22B

CD19-biotin + streptavidin conjugated PE

FIG.22C

FIG.22D

FIG.23A

FIG.23B

FIG.23C

FIG.24

EP 4 786 599 A1

FIG.25

## INTERNATIONAL SEARCH REPORT

International application No.

**PCT/CN2024/121364**

| A. CLASSIFICATION OF SUBJECT MATTER |
|---|
| C12N15/62(2006.01)i; C12N5/0783(2010.01)i; A61K35/17(2015.01)i; A61P35/00(2006.01)i; A61P37/00(2006.01)i |
| According to International Patent Classification (IPC) or to both national classification and IPC |

| B. FIELDS SEARCHED |
|---|
| Minimum documentation searched (classification system followed by classification symbols) |
| IPC: C12N, A61K, A61P |
| Documentation searched other than minimum documentation to the extent that such documents are included in the fields searched |
| Electronic data base consulted during the international search (name of data base and, where practicable, search terms used) |
| CNABS, DWPI, CNTXT, EPTXT, WOTXT, USTXT, CNKI, ISI web of science, baidu, Pubmed, STN on web, NCBI, 中文专利序列检索系统, Chinese Patents Sequence Search System, genbank, EMBL-EBI: 沛尔生技医药股份有限公司, 徐 浩, 跨膜结构域, transmembrane domain, TM, 细胞质结构域, cytoplasmic domain, CYP, CP, 胞内信号传导结构域, intracellular signaling domain, CSD, 自然杀伤细胞30, Nkp30, NCR3, DAP12, 41BB, 抗间皮素, mesothelin, CD19, CD22, 胞外茎, stalk, SEQ ID NOs. 79, 81, 83, 86, 87, 92 |

| C. DOCUMENTS CONSIDERED TO BE RELEVANT |
|---|

| Category* | Citation of document, with indication, where appropriate, of the relevant passages | Relevant to claim No. |
|---|---|---|
| X | WO 2022178367 A2 (UNIVERSITY OF SOUTHERN CALIFORNIA) 25 August 2022 (2022-08-25) page 7, paragraphs 36-38, page 8, paragraphs 41-43, pages 19-20, paragraph 100, pages 21-22, tables A1-2, page 54, paragraphs 149-150, and page 216, paragraph 539 | 1-15 |
| A | WO 2013033626 A2 (TRUSTEES OF DARTMOUTH COLLEGE) 07 March 2013 (2013-03-07) entire document | 1-15 |
| A | WO 2015142661 A1 (NOVARTIS AG et al.) 24 September 2015 (2015-09-24) entire document | 1-15 |
| A | WO 2016044605 A1 (BEATTY, G. et al.) 24 March 2016 (2016-03-24) entire document | 1-15 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **16 December 2024** | **26 December 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

<table>
<tr><td colspan="2">International application No.<br><br>**PCT/CN2024/121364**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | NG, Y. Y. et al. "T Cells Expressing NKG2D CAR with a DAP12 Signaling Domain Stimulate Lower Cytokine Production While Effective in Tumor Eradication" *Molecular Therapy*, Vol. 29, No. 1, 31 January 2021 (2021-01-31), pages 75-85 | 1-15 |
| A | MEMMER, S. et al. "The Stalk Domain of NKp30 Contributes to Ligand Binding and Signaling of a Preassembled NKp30-CD3ζ Complex" *The Journal of Biological Chemistry*, Vol. 291, No. 49, 17 October 2016 (2016-10-17), pages 25427-25438 | 1-15 |

Form PCT/ISA/210 (second sheet) (July 2022)

## INTERNATIONAL SEARCH REPORT

| International application No. |
| --- |
| **PCT/CN2024/121364** |

---

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.   ☑   forming part of the international application as filed.

     b.   ☐   furnished subsequent to the international filing date for the purposes of international search (Rule 13*ter*.1(a)),

           ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

---

Form PCT/ISA/210 (continuation of first sheet) (July 2022)

International application No.

**PCT/CN2024/121364**

| Patent document cited in search report | | | Publication date (day/month/year) | Patent family member(s) | | | Publication date (day/month/year) |
|---|---|---|---|---|---|---|---|
| WO | 2022178367 | A2 | 25 August 2022 | CA | 3208717 | A1 | 25 August 2022 |
| | | | | AU | 2022224066 | A1 | 07 September 2023 |
| | | | | IL | 305175 | A | 01 October 2023 |
| | | | | WO | 2022178367 | A3 | 29 September 2022 |
| | | | | US | 2024390496 | A1 | 28 November 2024 |
| | | | | KR | 20230153529 | A | 06 November 2023 |
| | | | | JP | 2024518011 | A | 24 April 2024 |
| | | | | EP | 4297769 | A2 | 03 January 2024 |
| WO | 2013033626 | A2 | 07 March 2013 | US | 2024200029 | A1 | 20 June 2024 |
| | | | | US | 2020360434 | A1 | 19 November 2020 |
| | | | | US | 11872248 | B2 | 16 January 2024 |
| | | | | US | 2015110760 | A1 | 23 April 2015 |
| | | | | US | 9833476 | B2 | 05 December 2017 |
| | | | | WO | 2013033626 | A3 | 02 May 2013 |
| | | | | US | 2018147240 | A1 | 31 May 2018 |
| | | | | US | 10682378 | B2 | 16 June 2020 |
| WO | 2015142661 | A1 | 24 September 2015 | EP | 3811970 | A1 | 28 April 2021 |
| | | | | EP | 3119425 | A1 | 25 January 2017 |
| | | | | EP | 3119425 | A4 | 22 November 2017 |
| | | | | EP | 3119425 | B1 | 23 September 2020 |
| | | | | US | 2021087279 | A1 | 25 March 2021 |
| | | | | US | 2017081411 | A1 | 23 March 2017 |
| | | | | ES | 2857226 | T3 | 28 September 2021 |
| WO | 2016044605 | A1 | 24 March 2016 | AU | 2024202889 | A1 | 23 May 2024 |
| | | | | BR | 112017005390 | A2 | 12 December 2017 |
| | | | | KR | 20230149327 | A | 26 October 2023 |
| | | | | KR | 20210149228 | A | 08 December 2021 |
| | | | | KR | 20170056622 | A | 23 May 2017 |
| | | | | KR | 102590396 | B1 | 19 October 2023 |
| | | | | CA | 2961636 | A1 | 24 March 2016 |
| | | | | JP | 2021097676 | A | 01 July 2021 |
| | | | | US | 2020377589 | A1 | 03 December 2020 |
| | | | | US | 11981731 | B2 | 14 May 2024 |
| | | | | MX | 2017003645 | A | 30 May 2017 |
| | | | | JP | 2023061944 | A | 02 May 2023 |
| | | | | ES | 2891332 | T3 | 27 January 2022 |
| | | | | EP | 3194443 | A1 | 26 July 2017 |
| | | | | EP | 3194443 | B1 | 14 July 2021 |
| | | | | MX | 2022012082 | A | 31 October 2022 |
| | | | | WO | 2016044605 | A8 | 06 May 2016 |
| | | | | EP | 3967709 | A1 | 16 March 2022 |
| | | | | AU | 2015317608 | A1 | 06 April 2017 |
| | | | | AU | 2015317608 | B2 | 11 March 2021 |
| | | | | US | 2017260268 | A1 | 14 September 2017 |
| | | | | US | 10577417 | B2 | 03 March 2020 |
| | | | | PL | 3194443 | T3 | 31 January 2022 |
| | | | | DK | 3194443 | T3 | 27 September 2021 |
| | | | | AU | 2021203790 | A1 | 08 July 2021 |
| | | | | AU | 2021203790 | B2 | 08 February 2024 |
| | | | | JP | 2017534261 | A | 24 November 2017 |

| International application No. |
| :--- |
| **PCT/CN2024/121364** |

| Patent document cited in search report | Publication date (day/month/year) | Patent family member(s) | | Publication date (day/month/year) |
| :--- | :--- | :--- | :--- | :--- |
| | | JP | 6839074 B2 | 03 March 2021 |
| | | EA | 201790624 A1 | 31 August 2017 |
| | | US | 2024317850 A1 | 26 September 2024 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Annals of Oncology*, vol. 32 (11), 1366-1380
- **GALLI E. et al.** *Br J Haematol.*, November 2023, vol. 203 (4), 564-570
- **KORELL F. et al.** *Med.*, 12 August 2022, vol. 3 (8), 538-564